(19) Europäisches Patentamt
European Patent Office
Office européen des brevets

(11) EP 1 715 887 B1

(12) **EUROPEAN PATENT SPECIFICATION**

(45) Date of publication and mention of the grant of the patent:
**20.06.2007 Bulletin 2007/25**

(21) Application number: **05702331.9**

(22) Date of filing: **24.01.2005**

(51) Int Cl.:
***A61K 38/27*** (2006.01) ***A61P 5/06*** (2006.01)

(86) International application number:
**PCT/IB2005/000171**

(87) International publication number:
**WO 2005/079838 (01.09.2005 Gazette 2005/35)**

# (54) N-TERMINALLY MONOPEGYLATED HUMAN GROWTH HORMONE CONJUGATES, PROCESS FOR THEIR PREPARATION, AND USE THEREOF

N-TERMINAL MONOPEGYLIERTE MENSCHLICHE WACHSTUMSHORMON-KONJUGATE, VERFAHREN ZU IHRER HERSTELLUNG UND IHRE ANWENDUNG

CONJUGUES DE L'HORMONE DE CROISSANCE HUMAINE MONOPEGYLES SUR LE PLAN N-TERMINAL, PROCEDE DE PREPARATION ET L'UTILISATION DESDITS CONJUGUES

(84) Designated Contracting States:
**AT BE BG CH CY CZ DE DK EE ES FI FR GB GR HU IE IS IT LI LT LU MC NL PL PT RO SE SI SK TR**

(30) Priority: **04.02.2004 US 771895**

(43) Date of publication of application:
**02.11.2006 Bulletin 2006/44**

(73) Proprietor: **Pharmacia Corporation**
**Peapack, NJ 07977 (US)**

(72) Inventor: **FINN, Rory, F.;**
**c/o Pfizer Global R. & D.**
**Chesterfield, MO63017-1732 (US)**

(74) Representative: **England, Peter Michael et al**
**Pfizer Limited**
**European Pharma Patent Department (I.P.C. 748)**
**Ramsgate Road**
**Sandwich**
**Kent CT13 9NJ (GB)**

(56) References cited:
**WO-A-03/044056** **US-A1- 2004 142 870**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 715 887 B1

SEQUENCE LISTING

<110> Pharmacia Corporation
Finn, Rory

<120> N-TERMINALLY MONOPEGYLATED HUMAN GROWTH HORMONE CONJUGATES, PROCESS FOR THEIR PREPARATION, AND METHODS OF USE THEREOF

<130> 32152A

<150> 10/771895
<151> 2004-02-04

<160> 1

<170> PatentIn version 3.2

<210> 1
<211> 191
<212> PRT
<213> Homo sapiens

<400> 1

```
Phe Pro Thr Ile Pro Leu Ser Arg Leu Phe Asp Asn Ala Met Leu Arg
1               5                   10                  15

Ala His Arg Leu His Gln Leu Ala Phe Asp Thr Tyr Gln Glu Phe Glu
            20                  25                  30

Glu Ala Tyr Ile Pro Lys Glu Gln Lys Tyr Ser Phe Leu Gln Asn Pro
        35                  40                  45

Gln Thr Ser Leu Cys Phe Ser Glu Ser Ile Pro Thr Pro Ser Asn Arg
    50                  55                  60

Glu Glu Thr Gln Gln Lys Ser Asn Leu Glu Leu Leu Arg Ile Ser Leu
65                  70                  75                  80

Leu Leu Ile Gln Ser Trp Leu Glu Pro Val Gln Ser Leu Arg Ser Val
                85                  90                  95

Phe Ala Asn Ser Leu Val Tyr Gly Ala Ser Asp Ser Asn Val Tyr Asp
            100                 105                 110

Leu Leu Lys Asp Leu Glu Glu Gly Ile Gln Thr Leu Met Gly Arg Leu
        115                 120                 125

Glu Asp Gly Ser Pro Arg Thr Gly Gln Ile Phe Lys Gln Thr Tyr Ser
    130                 135                 140

Lys Phe Asp Thr Asn Ser His Asn Asp Asp Ala Leu Leu Lys Asn Tyr
145                 150                 155                 160

Gly Leu Leu Tyr Cys Phe Arg Lys Asp Met Asp Lys Val Glu Thr Phe
                165                 170                 175

Leu Arg Ile Val Gln Cys Arg Ser Val Glu Gly Ser Cys Gly Phe
            180                 185                 190
```

## Description

The present application claims priority to US application N° 10/771895 filed February 04, 2004, which is incorporated by reference in its entirety as if written herein.

## FIELD OF THE INVENTION

The present application describes a chemical modification, including PEGylation, of human Growth Hormone (hGH) and agonist variants thereof by which the chemical and/or physiological properties of hGH can be changed. The PEGylated hGH may have an increased plasma residency duration, decreased clearance rate, improved stability, decreased antigenicity, decreased PEGylation heterogeneity or a combination thereof. The present application also describes processes for the modification of hGH. The present invention relates to pharmaceutical compositions comprising the modified hGH. A further embodiment is the use of the modified hGH for the treatment of growth and development disorders.

## BACKGROUND OF THE INVENTION

Human growth hormone (hGH) is a protein comprising a single chain of 191 amino acids cross-linked by two disulphide bridges and the monomeric form has a molecular weight of 22 kDa. Human GH is secreted by the pituitary gland and which also can be produced by recombinant genetic engineering. hGH will cause growth in all bodily tissues that are capable of growth. hGH plays an important role not only in promoting growth in the growing phase in human beings but also in maintaining normal body composition, anabolism, and lipid metabolism (K. Barneis. And U. Keller, Baillieres Clin. Endocrinlo. Metab. 10:337 (1996)).

Recombinant hGH has been commercially available for several years. Two types of therapeutically useful recombinant hGH preparations are present on the market: the authentic one, e.g. Genotropin™, or Nutropin™ and an analogue with an additional methionine residue at the N-terminal end, e.g. Somatonorm™. hGH is used to stimulate linear growth in patients with hypo pituitary dwarfism also referred to as Growth Hormone Deficiency (GHD) or Turner's syndrome but other indications have also been suggested including long-term treatment of growth failure in children who were born short for gestational age (SGA), for treatment of patients with Prader-Willi syndrome (PWS), chronic renal insufficiency (CRI), Aids wasting, and Aging. Adult GH deficiency (aGHD) patients have various problems, such as characteristic changes in body composition including increase in fat mass, decrease in lean body mass and extracellular fluid, and reduction of bone mineral density, metabolic abnormalities of lipids, and cardiovascular dysfunction. Many of those problems are improved by hGH replacement therapy (J. Verhelst J and R. Abs. Drugs.;62:2399 (2002).

A major biological effect of growth hormone (GH) is to promote growth in young mammals and maintenance of tissues in older mammals. The organ systems affected include the skeleton, connective tissue, muscles, and viscera such as liver, intestine, and kidneys. Growth hormones exert their effect through interaction with specific receptors on the target cell's membrane. hGH is a member of a family of homologous hormones that include placental lactogens, prolactins, and other genetic and species variants or growth hormone (Nicoll, C. S., et al. (1986) Endocrine Reviews 7: 169). hGH is unusual among these in that it exhibits broad species specificity and binds to either the cloned somatogenic (Leung, D. W., et al. [1987] Nature 330; 537) or prolactin receptor (Boutin, J. M., et al. [1988] Cell; 53: 69). The cloned gene for hGH has been expressed in a secreted form in Escherichia coli (Chang, C. N., et al. [1987] Gene 55:189), and its DNA and amino acid sequence has been reported (Goeddel, et al. [1979] Nature 281: 544; Gray, et al. [1985] Gene 39:247).

Human growth hormone (hGH) participates in much of the regulation of normal human growth and development. This pituitary hormone exhibits a multitude of biological effects including linear growth (somatogenesis), lactation, activation of macrophages, insulin-like and diabetogenic effects among others (Chawla, R, K. (1983) Ann. Rev. Med. 34, 519; Edwards, C. K. et al. (1988) Science 239, 769; Thomer, M. 0., et al. (1988) J. Clin. Invest. 81:745). Growth hormone deficiency in children leads to dwarfism, which has been successfully treated for more than a decade by exogenous administration of hGH.

In adults, as well as in children, hGH maintains a normal body composition by increasing nitrogen retention and stimulation of skeletal muscle growth, and by mobilization of body fat. Visceral adipose tissue is particularly responsive to hGH. In addition to enhanced lipolysis, hGH decreases the uptake of triglycerides into body fat stores. Serum concentrations of IGF-I (insulin-like growth factor-I), and IGFBP3 (insulin-like growth factor binding protein 3) are increased by hGH.

Human growth hormone (hGH) is a single-chain polypeptide consisting of 191 amino acids (molecular weight 21,500). Disulfide bonds link positions 53 and 165 and positions 182 and 189. Niall, Nature, New Biology, 230:90 (1971). hGH is a potent anabolic agent, especially due to retention of nitrogen, phosphorus, potassium, and calcium. Treatment of hypophysectomized rats with GH can restore at least a portion of the growth rate of the rats. Moore et al., Endocrinology

122:2920-2926 (1988). Among its most striking effects in hypo pituitary (GH-deficient) subjects is accelerated linear growth of bone-growth-plate-cartilage resulting in increased stature. Kaplan, *Growth Disorders in Children* and Adolescents (Springfield, IL: Charles C. Thomas, 1964).

hGH causes a variety of physiological and metabolic effects in various animal models including linear bone growth, lactation, activation of macrophages, insulin-like and diabetogenic effects, and others (R. K. Chawla et al., Annu. Rev. Med. 34:519 (1983); 0. G. P. Isaksson et al., Annu. Rev. Physiol. 47, 483 (1985); C. K. Edwards et al., Science 239, 769 (1988); M. 0. Thomer and M. L. Vance, J. Clin. Invest. 82:745 (1988); J. P. Hughes and H. G. Friesen, Ann. Rev. Physiol. 47:469 (1985)). It has been reported that, especially in women after menopause, GH secretion declines with age. Millard et al., Neurobiol. Aging, 11:229-235 (1990); Takahashi et al., Neuroendocrinology M, L6- 137-142 (1987). See also Rudman et al., J. Clin. Invest., 67:1361-1369 (1981) and Blackman, Endocrinology and Aging, 16:981 (1987). Moreover, a report exists that some of the manifestations of aging, including decreased lean body mass, expansion of adipose-tissue mass, and the thinning of the skin, can be reduced by GH treatment three times a week. See, e.g., Rudman et al., N. Eng. J. Med., 323:1-6 (1990) and the accompanying article in the same journal issue by Dr. Vance (pp. 52-54). These biological effects derive from the interaction between hGH and specific cellular receptors. Two different human receptors have been cloned, the hGH liver receptor (D. W. Leung et al., Nature 330:537(1987)) and the human prolactin receptor (J. M. Boutin et al., Mol. Endocrinology. 3:1455 (1989)). However, there are likely to be others including the human placental lactogen receptor (M. Freemark, M. Comer, G. Komer, and S. Handwerger, Endocrinol. 120:1865 (1987)). These homologous receptors contain a glycosylated extracellular hormone binding domain, a single transmembrane domain, and a cytoplasmic domain, which differs considerably in sequence and size. One or more receptors are assumed to play a determining role in the physiological response to hGH.

It is generally observed that physiologically active proteins administered into a body can show their pharmacological activity only for a short period of time due to their high clearance rate in the body. Furthermore, the relative hydrophobicity of these proteins may limit their stability and/or solubility.

For the purpose of decreasing the clearance rate, improving stability or abolishing antigenicity of therapeutic proteins, some methods have been proposed wherein the proteins are chemically modified with water-soluble polymers. Chemical modification of this type may block effectively a proteolytic enzyme from physical contact with the protein backbone itself, thus preventing degradation. Chemical attachment of certain water-soluble polymers may effectively reduce renal clearance due to increased hydrodynamic volume of the molecule. Additional advantages include, under certain circumstances, increasing the stability and circulation time of the therapeutic protein, increasing solubility, and decreasing immunogenicity. Poly(alkylene oxide), notably poly(ethylene glycol) (PEG), is one such chemical moiety that has been used in the preparation of therapeutic protein products (the verb "pegylate" meaning to attach at least one PEG molecule). The attachment of poly(ethylene glycol) has been shown to protect against proteolysis, Sada, et al., J. Fermentation Bioengineering 71: 137-139 (1991), and methods for attachment of certain poly(ethylene glycol) moieties are available. See U.S. Pat. No. 4,179,337, Davis et al., "Non-Immunogenic Polypeptides," issued Dec. 18, 1979; and U.S. Pat. No. 4,002,531, Royer, "Modifying Enzymes with Polyethylene Glycol and Product Produced Thereby," issued Jan. 11, 1977. For a review, see Abuchowski et al., in Enzymes as Drugs. (J. S. Holcerberg and J. Roberts, eds. pp. 367-383 (1981)) .

Other water-soluble polymers have been used, such as copolymers of ethylene glycol/propylene glycol, carboxymethylcellulose, dextran, poly(vinyl alcohol), poly(vinyl pyrrolidone), poly(-1,3-dioxolane), poly(-1,3,6-trioxane), ethylene/maleic anhydride copolymer, poly- amino acids (either homopolymers or random copolymers) .

A number of examples of pegylated therapeutic proteins have been described. ADAGEN®, a pegylated formulation of adenosine deaminase, is approved for treating severe combined immunodeficiency disease. ONCASPAR®, a pegylated L-asparaginase has been approved for treating hypersensitive ALL patients. Pegylated superoxide dismutase has been in clinical trials for treating head injury. Pegylated $\alpha$-interferon (U.S. 5,738,846, 5,382,657) has been approved for treating hepatitis; pegylated glucocerebrosidase and pegylated hemoglobin are reported to have been in preclinical testing. Another example is pegylated IL-6, EF 0 442 724, entitled, "Modified hIL-6," which discloses poly(ethylene glycol) molecules added to IL-6.

Another specific therapeutic protein, which has been chemically modified, is granulocyte colony stimulating factor, (G-CSF). G-CSF induces the rapid proliferation and release of neutrophilic granulocytes to the blood stream, and thereby provides therapeutic effect in fighting infection. European patent publication EP 0 401 384, published Dec. 12, 1990, entitled, "Chemically Modified Granulocyte Colony Stimulating Factor," describes materials and methods for preparing G-CSF to which poly(ethylene glycol) molecules are attached. Modified G-CSF and analogs thereof are also reported in EP 0 473 268, published Mar. 4, 1992, entitled "Continuous Release Pharmaceutical Compositions Comprising a Polypeptide Covalently Conjugated To A Water Soluble Polymer," stating the use of various G-CSF and derivatives covalently conjugated to a water soluble particle polymer, such as poly(ethylene glycol). A modified polypeptide having human granulocyte colony stimulating factor activity is reported in EP 0 335 423 published Oct. 4, 1989. Provided in U.S. 5,824,784 are methods for N-terminally modifying proteins or analogs thereof, and resultant compositions, including novel N-terminally chemically modified G-CSF compositions. U.S. 5,824,778 discloses chemically modified G-CSF.

For poly(ethylene glycol), a variety of means have been used to attach the poly(ethylene glycol) molecules to the protein. Generally, poly(ethylene glycol) molecules are connected to the protein via a reactive group found on the protein.

Amino groups, such as those on lysine residues or at the N-terminus, are convenient for such attachment. For example, Royer (U.S. Pat. No. 4,002,531, above) states that reductive alkylation was used for attachment of poly(ethylene glycol) molecules to an enzyme. Chamow et al., Bioconjugate Chem. 5: 133-140 (1994) report the modification of CD4 immunoadhesin with monomethoxypoly(ethylene glycol) aldehyde via reductive alkylation. The authors report that 50% of the CD4-Ig was MePEG-modified under conditions allowing control over the extent of pegylation. *Id.* at page 137. The authors also report that the in vitro binding capability of the modified CD4-Ig (to the protein gp 120) decreased at a rate correlated to the extent of MePEGylation Ibid. U.S. Pat. No. 4,904,584, Shaw, issued Feb. 27, 1990, relates to the modification of the number of lysine residues in proteins for the attachment of poly(ethylene glycol) molecules via reactive amine groups.

WO 93/00109 relates to a method for stimulating a mammal's or avian's GH responsive tissues comprising, maintaining a continuous, effective plasma GH concentration for a period of 3 or more days. One way of achieving such plasma concentration is stated to be by use of GH coupled to a macromolecular substance such as PEG (polyethylene glycol). The coupling to a macromolecular substance is stated to result in improved half-life. PEGylated human growth hormone has been reported in WO 93/00109 using mPEG aldehyde-5000 and mPEG N-hydroxysuccinmidyl ester (mPEG-NHS-5000). The use of mPEG-NHS resulted in heterogeneous mixtures of multiply PEGylated forms of hGH. WO 93/00109 also discloses the use of mPEG-maleimide to PEGylate cysteine hGH variants.

WO 99/03887 discloses a cysteine variant growth hormone that is PEGylated. Designated as BT-005, this conjugate is purported to be more effective at stimulating weight gain in growth hormone deficient rats and to have a longer half-life than hGH.

PEGylated human growth hormone has also been reported in Clark et al. using succinimidyl ester of carboxymethylated PEG (Journal of Biological Chemistry 271:21969-21977, 1996). Clark et al. describes derivates of hGH of increasing size using mPEG-NHS-5000, which selectively conjugates to primary amines. Increasing levels of PEG modification reduced the affinity for its receptor and increased the $EC_{50}$ in a cell-based assay up to 1500 fold. Olson et al., Polymer Preprints 38:568-569, 1997 discloses the use of N-hydroxysuccinimide (NHS)PEG and succinimidyl propionate (SPA)PEG to achieve multiply PEGylated hGH species.

WO 94/20069 prophetically discloses PEGylated hGH as part of a formulation for pulmonary delivery.

US 4,179,337 discloses methods of PEGylating enzymes and hormones to obtain physiologically active non-immunogenic, water-soluble polypeptide conjugates. GH is mentioned as one example of a hormone to be PEGylated.

EP 458064 A2 discloses PEGylation of introduced or naturally present cysteine residues in somatotropin. EP 458064 A2 further mentions the incorporation of two cysteine residues in a loop termed the omega loop stated to be located at residues 102-112 in wild type bovine somatotropin, more specifically EP 458064 A2 discloses the substitution of residues numbered 102 and 112 of bovine somatotropin from Ser to Cys and Tyr to Cys, respectively.

WO 95/11987 suggests attachment of PEG to the thio group of a cysteine residue being either present in the parent molecule or introduced by site directed mutagenesis. WO 95/11987 relates to PEGylation of protease nexin-1, however PEGylation in general of hGH and other proteins is suggested as well.

WO 99/03887 discloses, e.g., growth hormone modified by insertion of additional cys 25 serine residues and attachment of PEG to the introduced cysteine residues.

WO 00/42175 relates to a method for making proteins containing free cysteine residues for attachment of PEG. WO 00/42175 discloses the following muteins of hGH: T3C, S144C and T148C and the cysteine PEGylation thereof.

WO 97/11178 (as well as US 5849535, US 6004931, and US 6022711) relates to the use of GH variants as agonists or antagonists of hGH. WO 97/11178 also discloses PEGylation of hGH, including lysine PEGylation and the introduction or replacement of lysine (e.g. K168A and K172R) . WO 9711178 also discloses the substitution G120K.

WO 03/044056 discloses a variety of PEGylated hGH species including a branched 40K PEG aldehyde hGH conjugate.

The previous reports of PEGylated hGH require the attachment of multiple PEGs, which results in undesirable product heterogeneity, to achieve a hydrodynamic volume greater than the 70K molecular weight cut-off of the kidney filtration as described (Knauf, M.J. et al, *J. Biol. Chem.* 263: 15064-7.5070, 1988).

Currently administration of rhGH is daily for a long period of time, and therefore a less frequent administration would be highly desirable. A hGH molecule with a longer circulation half-life would decrease the number of necessary administrations and potentially provide more optimal therapeutic hGH levels with concomitant enhanced therapeutic effect.

Despite a number of attempts to PEGylate hGH, there is still an unmeet need for a PEGylated hGH molecule with the appropriate properties to be a viable commercial product. The present application describes PEG-hGH conjugates having a single PEG attached predominately at the N-terminal phenylalanine of hGH, which provides advantages over other PEG-hGH conjugates. The attachment of multiple low molecular weight (5Kd) PEGs at α- or ε-amino sites

(N-terminus and nine lysines in hGH) using mPEG aldehyde-5000 or mPEG N-hydroxysuccinmidyl ester (mPEG-NHS-5000) has been described in WO 93/00109, Clark et al. (Journal of Biological Chemistry 271:21969-21977, 1996, and Olson et al. (Polymer Preprints 38:568-569, 1997). This results in a heterogeneous population. As an illustration hGH with nine lysines may have some molecules having ten PEGs attached, some with nine, some with eight, some with seven, some with six, some with five, some with four, some with three, some with two, some with one and some with zero. And, among the molecules with several, the PEG may not be attached at the same location on different molecules. This resulting heterogeneity is disadvantageous when developing a therapeutic product making conjugation, purification, and characterization difficult, costly, and highly irreproducible. Another approach (WO 00/42175) has been to use hGH variants containing free cysteine residues for attachment of PEG. However, this approach can lead to incorrectly folded protein having incorrectly paired disulfide bonds and resulting in a heterogeneous PEGylated product that has the PEG attached at some or all of the cysteines. Having multiple PEGs attached to multiple sites may lead to molecules that have less stable bounds between the PEG and the various sites, which can become dissociated at different rates. This makes it difficult to accurately predict the pharmacokinetics of the product resulting in inaccurate dosing. A heterogeneous product also posses unwanted problems in obtaining regulatory approval for the therapeutic product.

Therefore, it would be desirable to have and utilise a PEGylated hGH molecule that has a single PEG attached at a single site. The present invention addresses these needs in a number of ways.

## SUMMARY OF THE INVENTION

The present invention relates to the use of chemically modified hGH and agonist variants thereof, which have at least one improved chemical or physiological property selected from but not limited to decreased clearance rate, increased plasma residency duration, increased stability, improved solubility, and decreased antigenicity. Thus, as described below in more detail, the present application has a number of aspects relating to chemically modifying polypeptides including but not limited to hGH and agonist variants thereof as well as specific modifications using a poly(ethylene glycol) butyraldehyde moiety.

The present application also describes methods of producing the chemically modified hGH and agonist variants thereof. Particularly, the present application describes a method of producing a chemically modified hGH using butyraldehyde, which results in greater N-terminal selectivity of attachment.

The present invention relates to compositions comprising the chemically modified hGH and agonist variants thereof, alone or in combination with another therapeutic agent.

The present invention also relates to the use of the chemically modified hGH and agonist variants thereof of the present invention, alone or in combination with another therapeutic agent, for the manufacture of a medicament for use in the prevention and/or treatment of disorders and/or diseases in which GH treatment is useful.

## BRIEF DESCRIPTION OF THE DRAWINGS

Figure 1 is a HPLC tracing of tryptic map analysis of the reaction of hGH and 40K branched butyrylaldehyde hGH or 40K branched aldehyde hGH. The top panel is the tryptic map of 40K Branched butyraldehyde hGH. The middle panel is the tryptic map of 40K Branched aldehyde hGH. The bottom panel is the tryptic map of unPEGylated hGH. T1 is the N-terminal tryptic fragment.

Figure 2 shows the amino acid sequence of human growth hormone (SEQ ID NO:1).

Figure 3 shows 40K Branched butyraldehyde hGH efficacy in Rat Weight Gain Assay. Hypophysectomized female Sprague-Dawley rats were purchased at the age of 4-5 weeks (100-125 g) from Harlan Labs. Upon entering the animal facilities, the animals were maintained at a constant room temperature of 80°F and weighed daily for 4-10 days in order to establish basal growth rates. Starting at day 0, rats (~100g) in control groups then received one daily subcutaneous injection of -0.3 mg/kg hGH (solid circles), or PBS (open circles), for eleven consecutive days. The 40K Branched butyraldehyde hGH test group (solid squares) received single doses of 1.8 mg/kg of PHA-794428 on days 0 and 6. There were 8-10 animals per group. Average growth +/- SEM is plotted.

Figure 4. shows the Dose-Responsive Growth Promoting Effects of 40K Branched butyraldehyde hGH in Rats. This efficacy study was performed in a manner similar to that described in Figure 3 except that a varied single dose of 40K Branched butyraldehyde hGH was administered (day 0, only) and the study ran for 6 days. Control groups received once-daily injections of either 0.3 mg/kg hGH (solid circles), or PBS vehicle (open circles) for six consecutive days. 40K Branched butyraldehyde hGH was dosed at 1.8 mg/kg (solid squares), 0.6 mg/kg (open squares), 0.2 mg/kg (solid triangles) or 0.067 mg/kg (open triangles). There were 8 animals per group.

Figure 5 shows tibial growth in response to 40K Branched butyraldehyde hGH. Hypophysectomized rats were treated as described in Figure 3. At day 11 animals were sacrificed, left tibias were removed and X-rayed and bone lengths were measured using a caliper. Average length +/- SEM is plotted. Asterisks denote significant differences from control group ($p<0.05$).

Figure 6 shows plasma IGF-1 levels for six-day efficacy study. Animals were treated as described Figure 4. Blood samples were taken at the various times and the serum IGF-1 levels determined by ELISA. Plotted are averages +/- SEM.

## DETAILED DESCRIPTION

hGH and agonist variants thereof are members of a family of recombinant proteins, described in US 4,658,021 (methionyl human growth hormone - Met-1-191 hGH) and US 5,633,352. Their recombinant production and methods of use are detailed in US 4,342,832, 4,601,980; US 4,898,830; US 5,424,199; and US 5,795,745.

Any purified and isolated hGH or agonist variant thereof, which is produced by host cells such as E. coli and animal cells transformed or transfected by using recombinant genetic techniques, may be used in the present invention. Additional hGH variants are described in US 6,143,523 and WO 92/09690 published Jun. 11, 1992. Among them, hGH or agonist variant thereof, which is produced by the transformed E. coli, is particularly preferable. Such hGH or agonist variant thereof may be obtained in large quantities with high purity and homogeneity. For example, the above hGH or agonist variant thereof may be prepared according to a method disclosed in US 4,342,832, 4,601,980; US 4,898,830; US 5,424,199; and US 5,795,745. The term "substantially has the following amino acid sequence" means that the above amino acid sequence may include one or more amino-acid changes (deletion, addition, insertion or replacement) as long as such changes will not cause any disadvantageous non-similarity in function to hGH or agonist variant thereof. It is more preferable to use the hGH or agonist variant thereof substantially having an amino acid sequence, in which at least one lysine, aspartic acid, glutamic acid, unpaired cysteine residue, a free N-terminal $\alpha$-amino group or a free C-terminal carboxyl group, is included.

The term "<u>hGH polypeptide or hGH protein</u>", when used herein, encompasses all hGH polypeptides, preferably from mammalian species, more preferably from human and murine species, as well as their variants, analogs, orthologs, homologs, and derivatives, and fragments thereof that are characterized by promoting growth in the growing phase and in maintaining normal body composition, anabolism, and lipid metabolism. Preferably, the term "hGH polypeptide or protein" refers to the hGH polypeptide of SEQ ID NO:1 as well as its variants, homologs and derivatives exhibiting essentially the same biological activity (promoting growth in the growing phase and in maintaining normal body composition, anabolism, and lipid metabolism). More preferably, the term "hGH polypeptide or protein" refers to the polypeptide of SEQ ID NO 1.

The term "hGH polypeptide variants", as used herein, refers to polypeptides from the same species but differing from a reference hGH polypeptide. Generally, differences are limited so that the amino acid sequences of the reference and the variant are closely similar overall and, in many regions, identical. Preferably, hGH polypeptides are at least 70%, 80%, 90%, 95%, 96%, 97%, 98% or 99% identical to a reference hGH polypeptide, preferably the hGH polypeptide of SEQ ID NO:1. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein.

Such hGH polypeptide variants may be naturally occurring variants, such as naturally occurring allelic variants encoded by one of several alternate forms of a hGH occupying a given locus on a chromosome of an organism, or isoforms encoded by naturally occurring splice variants originating from a single primary transcript. Alternatively, a hGH polypeptide variant may be a variant that is not known to occur naturally and that can be made using art-known mutagenesis techniques.

It is known in the art that one or more amino acids may be deleted from the N-terminus or C-terminus of a bioactive peptide or protein without substantial loss of biological function (see for instance, Ron et al., (1993), Biol Chem., 268 2984-2988 ; which disclosure is hereby incorporated by reference in its entirety).

It also will be recognized by one of ordinary skill in the art that some amino acid sequences of hGH polypeptides can be varied without significant effect of the structure or function of the protein. Such mutants include deletions, insertions, inversions, repeats, and substitutions selected according to general rules known in the art so as to have little effect on activity. For example, guidance concerning how to make phenotypically silent amino acid substitutions is provided in Bowie et al. (1990), Science 247:1306-1310, hereby incorporated by reference in its entirety, wherein the authors indicate that there are two main approaches for studying the tolerance of an amino acid sequence to change.

The first method relies on the process of evolution, in which mutations are either accepted or rejected by natural selection. The second approach uses genetic engineering to introduce amino acid changes at specific positions of a cloned hGH and selections or screens to identify sequences that maintain functionality. These studies have revealed

that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described in Bowie *et al.*, (1990) supra, and the references cited therein.

Typically seen as conservative substitutions are the replacements, one for another, among the aliphatic amino acids Ala, Val, Leu and Phe; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. In addition, the following groups of amino acids generally represent equivalent changes: (1) Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; (5) Phe, Tyr, Trp, His.

The term hGH polypeptide also encompasses all hGH polypeptides encoded by hGH analogs, orthologs, and/or species homologues. As used herein, the term "hGH analogs" refers to hGHs of different and unrelated organisms which perform the same functions in each organism but which did not originate from an ancestral structure that the organisms' ancestors had in common. Instead, analogous hGHs arose separately and then later evolved to perform the same function (or similar functions). In other words, analogous hGH polypeptides are polypeptides with quite different amino acid sequences but that perform the same biological activity, namely promoting growth in the growing phase and in maintaining normal body composition, anabolism, and lipid metabolism. As used herein, the term "hGH orthologs" refers to hGHs within two different species which sequences are related to each other via a common homologous hGH in an ancestral species but which have evolved to become different from each other. As used herein, the term "hGH homologs" refers to hGHs of different organisms which perform the same functions in each organism and which originate from an ancestral structure that the organisms' ancestors had in common. In other words, homologous hGH polypeptides are polypeptides with quite similar amino acid sequences that perform the same biological activity, namely promoting growth in the growing phase and in maintaining normal body composition, anabolism, and lipid metabolism. Preferably, hGH polypeptide homologs may be defined as polypeptides exhibiting at least 40%, 50%, 60%, 70%, 80%, 90%, 95%, 96%, 97%, 98% or 99% identity to a reference hGH polypeptide, preferably the hGH polypeptide of SEQ ID NO:1.

Thus, a hGH polypeptide according to the invention may be, for example: (i) one in which one or more of the amino acid residues are substituted with a conserved or non-conserved amino acid residue (preferably a conserved amino acid residue) and such substituted amino acid residue may or may not be one encoded by the genetic code: or (ii) one in which one or more of the amino acid residues includes a substituent group: or (iii) one in which the hGH polypeptide is fused with another compound, such as a compound to increase the half-life of the polypeptide (for example, polyethylene glycol): or (iv) one in which the additional amino acids are fused to the above form of the polypeptide, such as an IgG Fc fusion region peptide or leader or secretory sequence or a sequence which is employed for purification of the above form of the polypeptide or a pro-protein sequence.

hGH polypeptides may be monomers or multimers. Multimers may be dimers, trimers, tetramers or multimers comprising at least five monomeric polypeptide units. Multimers may also be homodimers or heterodimers. Multimers of the invention may be the result of hydrophobic, hydrophilic, ionic and/or covalent associations and/or may be indirectly linked, by for example, liposome formation. In one example, covalent associations are between the heterologous sequences contained in a fusion protein containing a hGH polypeptide or fragment thereof (see, e.g., US Patent Number 5,478,925, which disclosure is hereby incorporated by reference in its entirety). In another example, a hGH polypeptide or fragment thereof is joined to one or more polypeptides that may be either hGH polypeptides or heterologous polypeptides through peptide linkers such as those described in U.S. Pat. No. 5,073,627 (hereby incorporated by reference). Another method for preparing multimer hGH polypeptides involves use of hGH polypeptides fused to a leucine zipper or isoleucine zipper polypeptide sequence known to promote multimerization of the proteins in which they are found using techniques known to those skilled in the art including the teachings of WO 94/10308. In another example, hGH polypeptides may be associated by interactions between Flag® polypeptide sequence contained in fusion hGH polypeptides containing Flag® polypeptide sequence. hGH multimers may also be generated using chemical techniques known in the art such as cross-linking using linker molecules and linker molecule length optimization techniques known in the art (see, e.g., US 5,478,925), techniques known in the art to form one or more inter-molecule cross-links between the cysteine residues located within the sequence of the polypeptides desired to be contained in the multimer (see, e.g., US 5,478,925, addition of cysteine or biotin to the C terminus or N-terminus of hGH polypeptide and techniques to generate multimers containing one or more of these modified polypeptides (see, e.g., US 5,478,925), or any of the 30 techniques to generate liposomes containing hGH multimers (see, e.g., US Patent Number 5,478,925,), which disclosures are incorporated by reference in their entireties.

As used herein, the term "hGH polypeptide fragment" refers to any peptide or polypeptide comprising a contiguous span of a part of the amino acid sequence of a hGH polypeptide, preferably the polypeptide of SEQ ID NO:1.

More specifically, a hGH polypeptide fragment comprising at least 6, preferably at least 8 to 10, more preferably 12, 15, 20, 25, 30, 35, 40, 50, 60, 75, 100, 125, 150, 175, 191 consecutive amino acids of a hGH polypeptide according to the present invention. hGH polypeptide fragment may additionally be described as sub-genuses of hGH polypeptides

comprising at least 6 amino acids, wherein "at least 6" is defined as any integer between 6 and the integer representing the C-terminal amino acid of a hGH polypeptide including the polypeptide of SEQ ID NO:1. Further included are species of hGH polypeptide fragments at least 6 amino acids in length, as described above, that are further specified in terms of their N-terminal and C-terminal positions. Also encompassed by the term "hGH polypeptide fragment" as individual species are all hGH polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of the sequence listing or of the present invention is included in the present invention.

It is noted that the above species of polypeptide fragments of the present invention may alternatively be described by the formula "a to b"; where "a" equals the N-terminal most amino acid position and "b" equals the C-terminal most amino acid position of the polynucleotide; and further where "a" equals an integer between 1 and the number of amino acids of a hGH polypeptide sequence minus 6, and where "b" equals an integer between 7 and the number of amino acids of the hGH polypeptide sequence; and where "a" is an integer smaller then "b" by at least 6.

The above hGH polypeptide fragments can be immediately envisaged using the above description and are therefore not individually listed solely for the purpose of not unnecessarily lengthening the specification. Moreover, the above fragments do not necessarily need to have a hGH biological activity, although polypeptides having these activities are preferred embodiments of the invention, since they would be useful, for example, in immunoassays, in epitope mapping, epitope tagging, as vaccines, and as molecular weight markers. The above fragments may also be used to generate antibodies to a particular portion of the polypeptide.

Also encompassed by the term "hGH polypeptide fragment" are domains of hGH polypeptides. Such domains may eventually comprise linear or structural motifs and signatures including, but not limited to, leucine zippers, helix-turn-helix motifs, post-translational modification sites such as glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites. Such domains may present a particular biological activity such as DNA or RNA-binding, secretion of proteins, transcription regulation, enzymatic activity, substrate binding activity, etc...

A domain has a size generally comprised between 3 and 191 amino acids. In preferred embodiment, domains comprise a number of amino acids that is any integer between 6 and 191. Domains may be synthesized using any methods known to those skilled in the art, including those disclosed herein for the preparation of hGH polypeptides to produce anti-hGH antibodies. Methods for determining the amino acids that make up a domain with a particular biological activity include mutagenesis studies and assays to determine the biological activity to be tested.

Particularly preferred fragments in the context of the present invention are hGH polypeptides retaining a substantial biological activity, namely promoting growth in the growing phase and in maintaining normal body composition, anabolism, and lipid metabolism.

Alternatively, the polypeptides of the invention may be scanned for motifs, domains and/or signatures in databases using any computer method known to those skilled in the art. Searchable databases include Prosite (Hofmann et al., (1999) Nucl. Acids Res. 27:215-219; Bucher and Bairoch (1994) Proceedings 2nd International Conference on Intelligent Systems for Molecular Biology. Altman et al, Eds., pp53-61, AAAIPress, Menlo Park), Pfam (Sonnhammer et al., (1997) Proteins 28(3):405-20; Henikoff et al., (2000) Nucleic Acids Res. 28(1):228-30; Bateman et al., (2000) Nucleic Acids Res. 28(1):263-6), Blocks (Henikoff et al., (2000) Electrophoresis 21(9):1700-6), Print (Attwood et al., (1996) Nucleic Acids Res. 24(1):182-8), Prodom (Sonnhammer and Kahn (1994) Protein Sci. 3(3):482-92; Corpet et al. (2000) Nucleic Acids Res. 28(1):267-9), Sbase (Pongor et al. (1993) Protein Eng. 6(4):391-5; Murvai et al., (2000) Nucleic Acids Res. 28(1):260-2), Smart (Schultz et al., (1998) Proc. Natl. Acad. Sci. U S A 95, 5857-5864), Dali/FSSP (Holm and Sander (1996) Nucleic Acids Res. 24(1):206-9 ; Holm and Sander (1997) Nucleic Acids Res. 25(1):231-4 ; Holm and Sander (1999) Nucleic Acids Res. 27(1):244-7), HSSP (Sander and Schneider (1991) Proteins 9(1):56-68.), CATH (Orengo et al., (1997) Structure 5(8):1093-108; Pearl et al., (2000) Biochem. Soc. Trans. 28(2):269-75), SCOP (Murzin et al., (1995) J. Mol. Biol. 247(4):536-40; Lo Conte et al., (2000) Nucleic Acids Res. 28(1):257-9), COG (Tatusov et al., (1997) Science 278, 631 :637 ; Tatusov et al., (2000) Nucleic Acids Res. 28(1):33-6), specific family databases and derivatives thereof (Nevill-Manning et al., (1998) Proc. Natl. Acad. Sci. USA. 95, 5865-5871; Yona et al., (1999) Proteins 37(3):360-78; Attwood et al., (2000) Nucleic Acids Res. 28(1):225-7), each of which disclosures are hereby incorporated by reference in their entireties. For a review on available databases, see issue 1 of volume 28 of Nucleic Acid Research (2000), which disclosure is hereby incorporated by reference in its entirety.

The term "hGH polypeptide fragment" also encompasses epitopes-bearing fragments. These epitopes may be antigenic epitopes or both an antigenic epitope and an immunogenic epitope. An immunogenic epitope is defined as a part of a protein that elicits an antibody response *in vivo* when the polypeptide is the immunogen. On the other hand, a region of polypeptide to which an antibody binds is defined as an antigenic epitope. An epitope can comprise as few as 3 amino acids in a spatial conformation, which is unique to the epitope. Generally an epitope consists of at least 6 such amino acids, and more often at least 8-10 such amino acids.

A hGH epitope-bearing fragment according to the invention may be any fragment which length is between 6 amino acid and the full-length sequence of a hGH polypeptide, preferably a fragment between 6 and 50 amino acid. The epitope-bearing fragments may be specified by either the number of contiguous amino acid residues (as a sub-genus) or by specific N-terminal and C-terminal positions (as species) as described above.

Fragments which function as epitopes may be produced by any conventional means (*See, e.g.,* Houghten (1985), Proc. Natl. Acad. Sci. USA 82:5131-5135 and U.S. 4,631,21, which disclosures are hereby incorporated by reference in their entireties). Methods for determining the amino acids which make up an epitope include x-ray crystallography, 2-dimensional nuclear magnetic resonance, and epitope mapping, e.g., the Pepscan method described by Geysen et al., (1984), Proc. Natl. Acad. Sci. U.S.A. 81:3998-4002; PCT Publications WO 84/03564 and WO 84/03506, which disclosures are hereby incorporated by reference in their entireties. Another example is the algorithm of Jameson and Wolf, (1988), Comp. Appl. Biosci. 4:181-186 (said reference incorporated by reference in its entirety). The Jameson-Wolf antigenic analysis, for example, may be performed using the computer program PROTEAN, using default parameters (Version 4.0 Windows, DNASTAR, Inc.)

The present invention also provides for the exclusion of any hGH fragment species specified by N-terminal and C-terminal positions or of any fragment sub-genus specified by size in amino acid residues as described above. Any number of fragments specified by N-terminal and C-terminal positions or by size in amino acid residues as described above may be excluded as individual species. The present invention also provides for the exclusion of any hGH domain or epitope-bearing fragment in the same manner.

The hGH polypeptides of the present invention can be prepared in any suitable manner Such hGH polypeptides and fragments thereof may be purified from natural sources, chemically synthesized, produced by recombinant techniques including in vitro translation techniques or expression in a recombinant cell able to express hGH cDNA, or a combination of these methods, using techniques known to those skilled in the art (See, for example, "Methods in Enzymology, Academic Press, 1993" for a variety of methods for purifying proteins; Creighton, (1983) Proteins: Structures and Molecular Principles, W.H. Freeman & Co. 2nd Ed., T. E., New York; and Hunkapiller et al., (1984) Nature. 310 (5973): 105-11 for chemical synthesis of proteins and Davis et al. (1986) Basic Methods in Molecular Biology, ed., Elsevier Press, NY for recombinant techniques, which disclosures are incorporated by reference in their entireties). The polypeptides of the present invention are preferably provided in an isolated form, and may be partially or preferably substantially purified.

The terms "polynucleotides having at least x% identity with a polynucleotides of reference" and "polypeptides having at least x% identity with a polypeptide of reference" encompass polynucleotides or polypeptides which residue (nucleotide or amino acid respectively) sequence exhibit an identity percentage, as defined below, equal or superior to x compared to said reference polynucleotide or polypeptide sequence, respectively.

The identity percentage is determined after optimal alignment of two polynucleotides or polypeptide sequences over a comparison window, wherein portions of the polynucleotide or polypeptide sequences in the comparison window may comprise additions or deletions of one or more residue in order to optimize sequence alignment. The comparison window contains a certain number of positions (either a residue or a gap corresponding to an insertion/deletion of a residue), this number of positions corresponding to the window size. Each window position may present one of the following situations:

1°/ There is a residue (nucleotide or amino acid) on this position on the first aligned sequence and a different residue at the same position on the second aligned sequence, in other words the second sequence has a substituted residue at this position compared to the first sequence.
2°/ There is a residue (nucleotide or amino acid) on this position on the first aligned sequence and the same residue at the same position on the second aligned sequence.
3°/ There is a residue (nucleotide or amino acid) on this position on the first aligned sequence and no residue at the same position on the second aligned sequence, in other words the second sequence presents a deletion at this position compared to the first sequence.

The number of positions within the comparison window belonging to the first above-defined category is called R1.
The number of positions within the comparison window belonging to the second above-defined category is called R2.
The number of positions within the comparison window belonging to the third above-defined category is called R3.

The identity percentage (%id) is may be calculated by any of the following formulas:

$$\%id = R2/(R1+R2+R3) \times 100,$$

or

$$\%id=(R2+R3)/(R1+R2+R3)x100$$

Alignment of sequences to compare may be performed using any of the variety of sequence comparison algorithms and programs known in the art. Such algorithms and programs include, but are by no means limited to; TBLASTN, BLASTP, FASTA, TFASTA, , FASTDB, WU-BLAST, Gapped-BLAST, PSI-BLAST (Pearson and Lipman, (1988), Proc. Natl. Acad. Sci. USA 85:2444-2448; Altschul et al., (1990), J. Mol. Biol. 215:403-410; Altschul et al., (1993), Nature hGHtics 3:266-272; Altschul et al., (1997), Nuc. Acids Res. 25:3389-3402; Thompson et al., (1994), Nuc. Acids Res.. 22:4673-4680; Higgins et al., (1996), Meth. Enzymol. 266:383-402; Brutlag et al. (1990) Comp. App. Biosci. 6: 237-245; Jones and Swindells, (2002) . Trends Biochem Sci 27:161-4; Olsen et al. (1999) Pac Symp Biocomput; 302-13), the disclosures of which are incorporated by reference in their entireties.

In a particular embodiment, the Smith-Waterman method is used with scoring matrix such as PAM, PAM 250 or preferably with BLOSUM matrices such as BLOSUM60 or BLOSUM62 and with default parameters (Gap Opening Penalty=10 and Gap Extension Penalty=1) or with user-specified parameters preferably superior to default parameters.

In another particular embodiment, protein and nucleic acid sequences are aligned using the Basic Local Alignment Search Tool ("BLAST") programs with the default parameters or with modified parameters provided by the user. Preferably, the scoring matrix used is the BLOSUM62 matrix (Gonnet et al., (1992), Science 256:1443-1445; Henikoff and Henikoff, (1993), Proteins 17:49-61, which disclosures are hereby incorporated by reference in their entireties). Less preferably, the PAM or PAM250 matrices may also be used (see, e.g., Schwartz and Dayhoff, (1978), eds., Matrices for Detecting Distance Relationships: Atlas of Protein Sequence and Structure, Washington: National Biomedical Research Foundation, which disclosure is hereby incorporated by reference in its entirety).

In still another particular embodiment, polynucleotide or polypeptide sequences are aligned using the FASTDB computer program based on the algorithm of Brutlag *et al*. (1990), supra. Preferred parameters used in a FASTDB alignment of DNA sequences are:

> Matrix=Unitary, k-tuple=4, Mismatch Penalty= 1, Joining Penalty=30, Randomization Group Length=0, Cutoff Score= 1, Gap Penalty=5, Gap Size Penalty 0.05, Window Size=500 or the length of the subject nucleotide sequence, whichever is shorter. Preferred parameters used in a FASTDB amino acid alignment are: Matrix=PAM 0, k-tuple=2, Mismatch Penalty= 1, Joining Penalty=20, Randomization Group25Length=0, Cutoff Score= 1, Window Size=sequence length, Gap Penalty=5, Gap Size Penalty=0.05, Window Size=500 or the length of the subject amino acid sequence, whichever is shorter.

According to the present invention, poly(ethylene glycol) is covalently bound through amino acid residues of hGH or agonist variant thereof. A variety of activated poly(ethylene glycol)s having a number of different functional groups, linkers, configurations, and molecular weights are known to one skilled in the art, which may be used to create PEG-hGH conjugates or PEG-hGH agonist variant conjugates (for reviews see Roberts M.J. et al., Adv. Drug Del. Rev. 54:459-476, 2002), Harris J.M. et al., Drug Delivery Sytems 40:538-551, 2001) The present invention relates to a method of using aldehyde chemistry to direct selectivity of the PEG moiety to the N-terminus using a butyrylaldehyde linker moiety. The butyrylaldehyde linker results in increased N-terminal specificity compared to acetaldehyde linker (Table 1 and Figure 1)..

An embodiment of the present invention is a human growth hormone-PEG conjugate having the structure of Formula I or Formula II

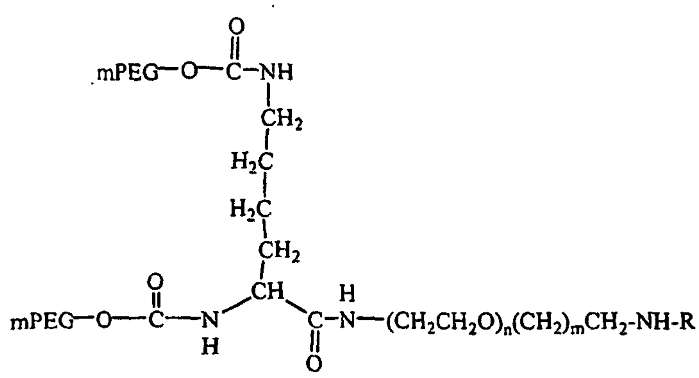

Formula I

or

$$mPEG\text{-}O(CH_2CH_2O)n(CH_2)_mCH_2\text{-}NH\text{-}R \qquad \text{Formula II}$$

wherein
n is an integer between 1 and 10;
m is an integer between 1 and 10;
R is human growth hormone, methionyl growth hormone or a human growth hormone variant.

In a particular embodiment n is between 1 and 5 and m is between 1 and 5.

In a particular embodiment of Formula I: n is 1 and m is 1; n is 1 and m is 2; n is 1 and m is 3; n is 1 and m is 4; n is 1 and m is 5; n is 1 and m is 6; n is 1 and m is 7; n is 1 and m is 8; n is 1 and m is 9; n is 1 and m is 10; n is 2 and m is 1; n is 2 and m is 2; n is 2 and m is 3; n is 2 and m is 4; n is 2 and m is 5; n is 2 and m is 6; n is 2 and m is 7; n is 2 and m is 8; n is 2 and m is 9; n is 2 and m is 10; n is 3 and m is 1; n is 3 and m is 2; n is 3 and m is 3; n is 3 and m is 4; n is 3 and m is 5; n is 3 and m is 6; n is 3 and m is 7; n is 3 and m is 8; n is 3 and m is 9; n is 3 and m is 10; n is 4 and m is 1; n is 4 and m is 2; n is 4 and m is 3; n is 4 and m is 4; n is 4 and m is 5; n is 4 and m is 6; n is 4 and m is 7; n is 4 and m is 8; n is 4 and m is 9; n is 4 and m is 10; n is 5 and m is 1; n is 5 and m is 2; n is 5 and m is 3; n is 5 and m is 4; n is 5 and m is 5; n is 5 and m is 6; n is 5 and m is 7; n is 5 and m is 8; n is 5 and m is 9; n is 5 and m is 10; n is 6 and m is 1; n is 6 and m is 2; n is 6 and m is 3; n is 6 and m is 4; n is 6 and m is 5; n is 6 and m is 6; n is 6 and m is 7; n is 6 and m is 8; n is 6 and m is 9; n is 7 and m is 10; n is 7 and m is 1; n is 7 and m is 2; n is 7 and m is 3; n is 7 and m is 4; n is 7 and m is 5; n is 7 and m is 6; n is 7 and m is 7; n is 7 and m is 8; n is 7 and m is 9; n is 7 and m is 10; n is 8 and m is 1; n is 8 and m is 2; n is 8 and m is 3; n is 8 and m is 4; n is 8 and m is 5; n is 8 and m is 6; n is 8 and m is 7; n is 8 and m is 8; n is 8 and m is 9; n is 8 and m is 10; n is 9 and m is 1; n is 9 and m is 2; n is 9' and m is 3; n is 9 and m is 4; n is 9 and m is 5; n is 9 and m is 6; n is 9 and m is 7; n is 9 and m is 8; n is 9 and m is 9; n is 9 and m is 10; n is 10 and m is 1; n is 10 and m is 2; n is 10 and m is 3; n is 10 and m is 4; n is 10 and m is 5; n is 10 and m is 6; n is 10 and m is 7; n is 10 and m is 8; n is 10 and m is 9; n is 10 and m is 10.

A specific embodiment is a human growth hormone-PEG conjugate having the structure:

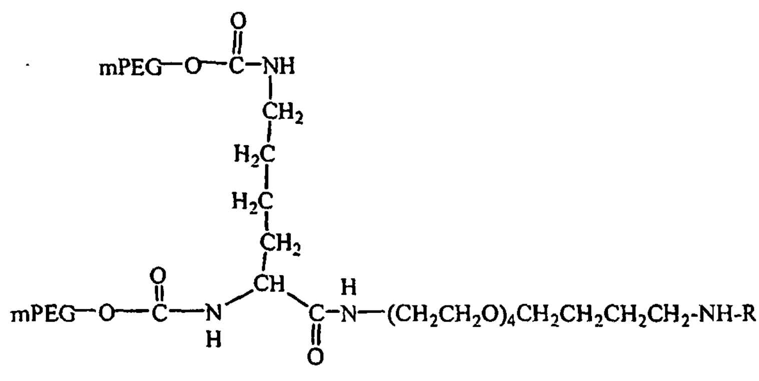

or

$$mPEG\text{-}O(CH_2CH_2O)_4CH_2CH_2CH_2NH\text{-}R$$

wherein R is human growth hormone, methionyl human growth hormone or a human growth hormone variant.

Another specific embodiment of the present invention human growth hormone-PEG conjugate wherein the human growth hormone comprises or consists of the amino acid sequence of SEQ ID NO: 1.

The present application describes a human growth hormone-PBG conjugate wherein greater than 80%, more preferably 81%, more preferably 82%, more preferably 83%, more preferably 84%, more preferably 85%, more preferably 86%, more preferably 87%, more preferably 88%, more preferably 89%, more preferably 90%, more preferably 91%, more preferably 92%, more preferably 93%, more preferably 94%, more preferably 95%, more preferably 96%, more preferably 97, and more preferably 98% of the polyethylene glycol is conjugated to the amino-terminal phenylalanine of the amino acid sequence of SEQ ID NO:1.

A specific embodiment of the present invention is use of a human growth hormone-PEG conjugate wherein greater than 90% of the polyethylene glycol is conjugated to the amino-terminal phenylalanine of the amino acid sequence of SEQ ID NO:1.

Another specific embodiment of the present invention is use of a human growth hormone-PEG conjugate wherein greater than 95% of the polyethylene glycol is conjugated to the amino-terminal phenylalanine of the amino acid sequence of SEQ ID NO:1.

The poly (ethylene glycol) used in the present invention is not restricted to any particular form or molecular weight range. The poly(ethylene glycol) molecular weight may between about 500 and about 100,000 Dalton. The term "about" indicating that in preparations of polyethylene glycol; some molecules will weigh more, some less, than the stated molecular weight and the stated molecular weight refers to the average molecular weight. It is understood that there is some degree of polydispersity associated with polymers such as poly(ethylene glycol). It is preferable to use PEGs with low polydispersity. Normally, a PEG with molecular weight of about 500 to about 60,000 is used. A specific PEG molecular weight range, of the present invention is from about 1,000 to about 40,000. In another specific embodiment the PEG molecular weight is greater than about 5,000 to about 40,000. In another specific embodiment the PEG molecular weight about 20,000 to about 40,000. Other sizes may be used, depending on the desired therapeutic profile (e.g. duration of sustained release desired, the effects, if any on biological activity, the degree or lack of antigenicity and other known effects of the polyethylene to a therapeutic protein. For example the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 45,000, 50,000, 55,000, 60,000, 65,000, 70,000, 75,000, 80,000, 85,000, 90,000, 95,000, or 100,000 Dalton.

In another embodiment the poly(ethylene glycol) is a branched PEG having more than one PEG moiety attached (see U.S. 5,932,462; U.S. 5,342,940; U.S. 5,643,575; U.S. 5,919,455; U.S. 6,113,906; U.S. 5,183,660; Kodera Y., Bioconjugate Chemistry 5:283-288 (1994); and WO 02/09766. In a preferred embodiment the molecular weight of each poly(ethylene glycol) of the branched PEG is about 5,000-20,000. In a specific embodiment the molecular weight of each poly(ethylene glycol) of the branched PEG is about 20,000.

Poly(alkylene oxide) s, notably poly(ethylene glycol)s, are bound to hGH or agonist variant thereof via a terminal reactive group, which may or may not leave a linking moiety (spacer) between the PEG and the protein. In order to form the hGH conjugates or agonist variant thereof of the present invention, polymers such as poly(alkylene oxide) are converted into activated forms, as such term is known to those of ordinary skill in the art. The reactive group, for example, is a terminal reactive group, which mediates a bond between chemical moieties on the protein and poly(ethylene glycol). Typically, one or both of the terminal polymer hydroxyl end-groups, (i.e. the alpha and omega terminal hydroxyl groups) are converted into reactive functional groups, which allows covalent conjugation. This process is frequently referred to as "activation" and the poly(ethylene glycol) product having the reactive group is hereinafter referred to as "an activated poly(ethylene glycol)". In a specific embodiment one of the terminal polymer hydroxyl end-groups is converted or capped with a non-reactive group. In a specific embodiment one of the terminal polymer hydroxyl end-groups is converted or capped with a methyl group. As used herein, the term "mPEG" refers to a PEG, which is capped at one end with a methyl group. The mPEG can be represented structurally as

$$CH_3O\text{-}(CH_2CH_2O)_n\text{-}H$$

Polymers containing both $\alpha$ and $\varepsilon$ linking groups are referred to as "bis-activated poly(alkylene oxides)" and are referred to as "bifunctional". Polymers containing the same reactive group on $\alpha$ and $\varepsilon$ terminal hydroxyls are sometimes referred to as "homobifunctional" or "homobis-activated". Polymers containing different reactive groups on $\alpha$ and $\varepsilon$ terminal hydroxyls are sometimes referred to as "heterobifunctional" (see for example WO 01/26692) or "heterobis-activated". Polymers containing a single reactive group are referred to as "mono-activated" polyalkylene oxides or "monofunctional". Other substantially non-antigenic polymers are similarly "activated" or "functionalized".

The activated polymers are thus suitable for mediating a bond between chemical moieties on the protein, such as α- or ε-amino, carboxyl or thiol groups, and poly(ethylene glycol). Bis-activated polymers can react in this manner with two protein molecules or one protein molecule and a reactive small molecule in another embodiment to effectively form protein polymers or protein-small molecule conjugates through cross linkages.

In one preferred embodiment of the invention secondary amine or amide linkages are formed using the N-terminal α-amino group or ε-amino groups of lysine of hGH or agonist variant thereof and the activated PEG. In another preferred aspect of the invention, a secondary amine linkage is formed between the N-terminal primary α- or ε-amino group of hGH or agonist variant thereof and single or branched chain PEG aldehyde by reductive alkylation with a suitable reducing agent such as $NaCNBH_3$, $NaBH_3$, Pyridine Borane etc. as described in Chamow et al., Bioconjugate Chem. 5: 133-140 (1994), US Pat No. 4,002,531, WO 90/05534, and US Pat. No 5,824,784.

In a preferred embodiment at least 70%, preferably at least 80%, preferably at least 81%, preferably at least 82%, preferably at least 83%, preferably at least 84%, preferably at least 85%, preferably at least 86%, preferably at least 81%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, and most preferably at least 98% of the poly(ethylene glycol) is on the amino terminal α-amino group.

Conjugation reactions, referred to as pegylation reactions, were historically carried out in solution with molar excess of polymer and without regard to where the polymer will attach to the protein. Such general techniques, however, have typically been proven inadequate for conjugating bioactive proteins to non-antigenic polymers while retaining sufficient bioactivity. One way to maintain the hGH or agonist variant thereof bioactivity is to substantially avoid the conjugation of those hGH or agonist variant thereof reactive groups associated with the receptor binding site(s) in the polymer coupling process. Another aspect of the present application is to provide a process of conjugating poly(ethylene glycol) to hGH or agonist variant thereof maintaining high levels of retained activity.

The chemical modification through a covalent bond may be performed under any suitable condition generally adopted in a reaction of a biologically active substance with the activated poly(ethylene glycol). The conjugation reaction is carried out under relatively mild conditions to avoid inactivating the hGH or agonist variant thereof. Mild conditions include maintaining the pH of the reaction solution in the range of 3 to 10 and the reaction temperatures within the range of from about 0°-37°C. In the cases where the reactive amino acid residues in hGH or agonist variant thereof have free amino groups, the above modification is preferably carried out in a non-limiting list of suitable buffers (pH 3 to 10), including phosphate, MES, citrate, acetate, succinate or HEPES, for 1-48 hrs at 4°-37°C. In targeting N-terminal amino groups with reagents such as PEG aldehydes pH 4-7 is preferably maintained. The activated poly(ethylene glycol) may be used in about 0.01-100 times, preferably about 0.01-2.5 times, the molar amount of the number of free amino groups of hGH or agonist variant thereof. On the other hand, where reactive amino acid residues in hGH or agonist variant thereof have the free carboxyl groups, the above modification is preferably carried out in pH from about 3.5 to about 5.5, for example, the modification with poly(oxyethylenediamine) is carried out in the presence of carbodiimide (pH 4-5) for 1-24 hrs at 4°-37°C. The activated poly(ethylene glycol) may be used in 0.01-300 times the molar amount of the number of free carboxyl groups of hGH or agonist variant thereof.

In separate embodiments, the upper limit for the amount of polymer included in the conjugation reactions exceeds about 1:1 to the extent that it is possible to react the activated polymer and hGH or agonist variant thereof without forming a substantial amount of high molecular weight species, i.e. more than about 20% of the conjugates containing more than about one strand of polymer per molecule of hGH or agonist variant thereof. For example, it is contemplated in this aspect of the invention that ratios of up to about 6:1 can be employed to form significant amounts of the desired conjugates which can thereafter be isolated from any high molecular weight species.

In another aspect of this application, bifunctionally activated PEG derivatives may be used to generate polymeric hGH or agonist variant thereof-PEG molecules in which multiple hGH or agonist variant thereof molecules are crosslinked via PEG. Although the reaction conditions described herein can result in significant amounts of unmodified hGH or agonist variant thereof, the unmodified hGH or agonist variant thereof can be readily recycled into future batches for additional conjugation reactions. The processes of the present application generate surprisingly very little, *i.e.* less than about 30% and more preferably; less than about 10%, of high molecular weight species and species containing more than one polymer strand per hGH or agonist variant thereof. These reaction conditions are to be contrasted with those typically used for polymeric conjugation reactions wherein the activated polymer is present in several-fold molar excesses with respect to the target. In other aspects of the application, the polymer is present in amounts of from about 0.1 to about 50 equivalents per equivalent of hGH or agonist variant thereof. In other aspects of the application, the polymer is present in amounts of from about 1 to about 10 equivalents per equivalent of hGH or agonist variant thereof.

The conjugation reactions of the present application initially provide a reaction mixture or pool containing mono- and di-PEG-hGH conjugates, unreacted hGH, unreacted polymer, and usually less than about 20% high molecular weight species. The high molecular weight species include conjugates containing more than one polymer strand and/or

polymerized PEG-hGH or agonist variant thereof species. After the unreacted species and high molecular weight species have been removed, compositions containing primarily mono- and di-polymer-hGH or agonist variant thereof conjugates are recovered. Given the fact that the conjugates for the most part include a single polymer strand, the conjugates are substantially homogeneous. These modified hGH or agonist variant thereof have at least about 0.1% of the in vitro biological activity associated with the native or unmodified hGH or agonist variant thereof as measured using standard FDC-P1 cell proliferation assays, (Clark et al. Journal of Biological Chemistry 271:21969-21977, 1996), receptor binding assay (US 5,057,417), or hypophysectomized rat growth (Clark et al. Journal of Biological Chemistry 271:21969-21977, 1996). preferred aspects of the application, however, the modified hGH or agonist variant thereof have about 25% of the in vitro biological activity, more preferably, the modified hGH or agonist variant thereof have about 50% of the *in vitro* biological activity, more preferably, the modified hGH or agonist variant thereof have about 75% of the in vitro biological activity, and most preferably the modified hGH or agonist variant thereof have equivalent or improved in vitro biological activity.

The processes of the present application preferably include rather limited ratios of polymer to hGH or agonist variant thereof. Thus, the hGH or agonist variant thereof conjugates have been found to be predominantly limited to species containing only one strand of polymer. Furthermore, the attachment of the polymer to the hGH or agonist variant thereof reactive groups is substantially less random than when higher molar excesses of polymer linker are used. The unmodified hGH or agonist variant thereof present in the reaction pool, after the conjugation reaction has been quenched, can be recycled into future reactions using ion exchange or size exclusion chromatography or similar separation techniques.

A poly (ethylene glycol)-modified hGH or agonist variant thereof, namely chemically modified protein according to the present application, may be purified from a reaction mixture by conventional methods which are used for purification of proteins, such as dialysis, salting-out, ultrafiltration, ion-exchange chromatography, hydrophobic interaction chromatography (HIC), gel chromatography and electrophoresis. Ion-exchange chromatography is particularly effective in removing unreacted poly(ethylene glycol) and hGH or agonist variant thereof. In a further embodiment of the application, the mono- and di-polymer-hGH or agonist variant thereof species are isolated from the reaction mixture to remove high molecular weight species, and unmodified hGH or agonist variant thereof. Separation is effected by placing the mixed species in a buffer solution containing from about 0.5-10 mg/mL of the hGH or agonist variant thereof- polymer conjugates. Suitable solutions have a pH from about 4 to about 10. The solutions preferably contain one or more buffer salts selected from KCl, NaCl, $K_2HPO_4$, $KH_2PO_4$, $Na_2HPO_4$, $NaH_2PO_4$, $NaHCO_3$, $NaBO_4$, $CH_3CO_2H$, and NaOH.

Depending upon the reaction buffer, the hGH or agonist variant thereof polymer conjugate solution may first have to undergo buffer exchange/ultrafiltration to remove any unreacted polymer. For example, the PEG-hGH or agonist variant thereof conjugate solution can be ultrafiltered across a low molecular weight cut-off (10,000 to 30,000 Dalton) membrane to remove most unwanted materials such as unreacted polymer, surfactants, if present, or the like.

The fractionation of the conjugates into a pool containing the desired species is preferably carried out using an ion exchange chromatography medium. Such media are capable of selectively binding PEG-hGH or agonist variant thereof conjugates via differences in charge, which vary in a somewhat predictable fashion. For example, the surface charge of hGH or agonist variant thereof is determined by the number of available charged groups on the surface of the protein. These charged groups typically serve as the point of potential attachment of poly(alkylene oxide) polymers. Therefore, hGH or agonist variant thereof conjugates will have a different charge from the other species to allow selective isolation.

Strongly polar anion or cation exchange resins such as quaternary amine or sulfopropyl resins, respectively, are used for the method of the present application. Ion exchange resins are especially preferred. A non-limiting list of included commercially available cation exchange resins suitable for use with the present invention are SP-hitrap®, SP Sepharose HP® and SP Sepharose® fast flow. Other suitable cation exchange resins e.g. S and CM resins can also be used. A non-limiting list of anion exchange resins, including commercially available anion exchange resins, suitable for use with the present invention are Q-hitrap®, Q Sepharose HP®, and Q sepharose® fast flow. Other suitable anion exchange resins, e.g. DEAB resins, can also be used.

For example, the anion or cation exchange resin is preferably packed in a column and equilibrated by conventional means. A buffer having the same pH and osmolality as the polymer conjugated hGH or agonist variant thereof solution is used. The elution buffer preferably contains one or more salts selected from KCl, NaCl, $K_2HPO_4$, $KH_2PO_4$, $Na_2HPO_4$, $NaH_2PO_4$, $NaHCO_3$, $NaBO_4$, and $(NH_4)_2CO_3$. The conjugate-containing solution is then adsorbed onto the column with unreacted polymer and some high molecular weight species not being retained. At the completion of the loading, a gradient flow of an elution buffer with increasing salt concentrations is applied to the column to elute the desired fraction of polyalkylene oxide-conjugated hGH or agonist variant thereof. The eluted pooled fractions are preferably limited to uniform polymer conjugates after the cation or anion exchange separation step. Any unconjugated hGH or agonist variant thereof species can then be back washed from the column by conventional techniques. If desired, mono and multiply pegylated hGH or agonist variant thereof species can be further separated from each other via additional ion exchange chromatography or size exclusion chromatography.

Techniques utilizing multiple isocratic steps of increasing concentration of salt or pH can also be used. Multiple isocratic elution steps of increasing concentration will result in the sequential elution of di- and then mono-hGH or agonist variant thereof-polymer conjugates.

The temperature range for elution is between about 4°C and about 25°C. Preferably, elution is carried out at a temperature of from about 4°C to about 22°C. For example, the elution of the PEG-hGH or agonist variant thereof fraction is detected by UV absorbance at 280 nm. Fraction collection may be achieved through simple time elution profiles.

A surfactant can be used in the processes of conjugating the poly(ethylene glycol) polymer with the hGH or agonist variant thereof moiety. Suitable surfactants include ionic-type agents such as sodium dodecyl sulfate (SDS). Other ionic surfactants such as lithium dodecyl sulfate, quaternary ammonium compounds, taurocholic acid, caprylic acid, decane sulfonic acid, etc. can also be used. Non-ionic surfactants can also be used. For example, materials such as poly(oxyethylene) sorbitans (Tweens), poly(oxyethylene) ethers (Tritons) can be used. See also Neugebauer, A Guide to the Properties and Uses of Detergents in Biology and Biochemistry (1992) Calbiochem Corp. The only limitations on the surfactants used in the processes described herein are that they are used under conditions and at concentrations that do not cause substantial irreversible denaturation of the hGH or agonist variant thereof and do not completely inhibit polymer conjugation. The surfactants are present in the reaction mixtures in amounts from about 0.01-0.5%; preferably from 0.05-0.5%; and most preferably from about 0.075-0.25%. Mixtures of the surfactants are also contemplated.

It is thought that the surfactants provide a temporary, reversible protecting system during the polymer conjugation process. Surfactants have been shown to be effective in selectively discouraging polymer conjugation while allowing lysine-based or amino terminal-based conjugation to proceed.

The present poly(ethylene glycol) -modified hGH or agonist variant thereof has a more enduring pharmacological effect, which may be possibly attributed to its prolonged half-life *in vivo*.

Another embodiment of the application relates to methods for the prevention and/or treatment of a disease or disorder in which use of GH, preferably hGH is beneficial, comprising administering to a patient in need thereof a therapeutically effective amount of a poly(ethylene glycol)-modified hGH of the invention or agonist variant thereof, alone or in combination with another therapeutic agent. The present invention specifically relates to the use of a poly(ethylene glycol)-modified hGH of the invention or agonist variant thereof in the manufacture of a medicament for the prevention and/or treatment of a disease or disorder in which use of GH, preferably hGH is beneficial. In addition, the invention also relates to a pharmaceutical composition comprising a poly(ethylene glycol)-modified hGH of the invention or agonist variant thereof for the prevention and/or treatment of a disease or disorder in which use of GH, preferably hGH is beneficial.

Diseases or disorders in which the use of GH is beneficial include, but are limited to, growth hormone deficiency (GHD), adult growth hormone deficiency (aGHD), Turner's syndrome, growth failure in children who were born short for gestational age (SGA), Prader-Willi syndrome (PWS); chronic renal insufficiency (CRI), Aids wasting, Aging, end-stage Renal Failure, Cystic Fibrosis, Erectile dysfunction, HIV lipodystrophy, Fibromyalgia, Osteoporosis, Memory disorders, Depression, Crohn's disease, Skeletal dysplasias, Traumatic brain injury, Subarachnoid haemorrhage, Noonan's syndrome. Down's syndrome, Idiopathic short stature (ISS), End stage renal disease (ESRD), Very low birth weight (VLBW), Bone marrow stem cell rescue, Metabolic syndrome, Glucocorticoid myopathy, Short stature due to glucocorticoid treatment in children, and Failure of growth catching for short premature children.

In a more specific embodiment of the invention, the poly(ethylene glycol)-modified hGH of the application or agonist variants thereof are used in the prevention and/or treatment of a disorders or diseases selected from the group consisting of idiopathic short stature, very low birth weight, traumatic brain injury, metabolic syndrome, subarachnoid haemorrhage, short stature due to glucocorticoid treatment in children; failure of growth catching for short premature children, and Noorian's syndrome. application

Another embodiment of the invention relate to pharmaceutical compositions comprising a poly(ethylene glycol)-modified hGH of the invention or agonist variant thereof, alone or in combination with another therapeutic agent, and at least one pharmaceutically acceptable excipient or carrier. The present poly(ethylene glycol)-modified hGH or agonist variant thereof may then be formulated into pharmaceuticals containing also a pharmaceutically acceptable diluent, an agent for preparing an isotonic solution, a pH-conditioner and the like in order to administer them into a patient.

The above pharmaceuticals may be administered subcutaneously, intramuscularly, intravenously, pulmonary, intradermally, or orally, depending on a purpose of treatment. A dose may be also based on the kind and condition of the disorder of a patient to be treated, being normally between 0.1 mg and 5 mg by injection and between 0.1 mg and 50 mg in an oral administration for an adult.

As used herein, the poly(ethylene glycol)-modified hGH or agonist variants thereof of the present invention may be used in combination with another therapeutic agent. As used herein, the terms "co-administration". "co-administered" and "in combination with", referring to the compounds A and one or more other therapeutic agents, is intended to mean, and does refer ' to and include the following:

- simultaneous administration of such combination of A and therapeutic agent(s) to a patient in need of treatment, when such components are formulated together into a single dosage form which releases said components at

substantially the same time to said patient,

- substantially simultaneous administration of such combination of A and therapeutic agent(s) to a patient in need of treatment, when such components are formulated apart from each other into separate dosage forms which are taken at substantially the same time by said patient, whereupon said components are released at substantially the same time to said patient
- sequential administration of such combination of A and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of A and therapeutic agent(s) to a patient in need of treatment, when such components are formulated together into a single dosage form which releases said components in a controlled manner whereupon they are concurrently, consecutively, and/or overlappingly administered at the same and/or different times by said patient.

Suitable examples of other therapeutic agents which may be used in combination with A, their pharmaceutically acceptable salts and/or their derived forms include, but are by no mean limited to: aromatase inhibitors such as exemestane, formestane, atamestane, fadrozole, letrozole, vorozole and anastrozole; free fatty acid regulators including fibric acid derivatives (such as fenofibrate, clofibrate, gemfibrozil, bezafibrate and ciprofibrate) and nicotinic acid derivatives such as acipimox; insulin sensitizing agents including but not limited to biguanides such as metformin, PPAR gamma insulin sensitizing agents and thiazolodeniones such as troglitazone and rosiglitazone Troglitazone, 5-[[4-[3,4-Dihydro-6-hydroxy-2,5,7,8-tetramethyl-2H-]-benzopyran-2-yl) methoxy]phenyl]methyl3 -2,4-thiazolidinedione V411(DIABII, Glaucanin) Pioglitazone (ACTOS, AD 4833, U 72107, U 72107A, U 72107E, ZACTOS) Chemical Name: 2,4-Thiazolidinedione, 5-[[4-[2-(5-ethyl-2-pyridinyl) ethoxy]phenyl]methyl] -, monohydrochloride, (a/-); Rosiglitazone (Avandia, BRL 49653, BRL 49653C) Chemical Name: 2,4 Thiazolidinedione, 5-[[4-[2- (methyl-2-pyridinylarnino)ethoxy]phenyl]methyl]; 25 Bexarotene-oral (LGD 1069 oral, Targretin oral, Targretin, Targretyn oral Targrexin oral) Chemical Name: 4-[1-(3,5,5,8,8-Pentamethyl-5,6,7,8-tetrahydro-2-naphthyl) ethenyl]benzoic acid; ZD 2079, (ICI D 2079) (Chemical Name:R)-N-[2-4-(Carboxymethyl) 30 phenoxy]ethyl)-N-(2-hydroxy-2-phenethyl) ammonium chloride: Netoglitazone, (Isaglitazone, MCC 555, RWJ 241947) (Chemical Name: 5-[6(2-Fluorobenzyloxy)naphthalen-2-ylmethyl]thiazolidine-2,4-dione) ; INS (D-chiro-inositol) (Chemical Name: D-1,2,3,4,5,6- Hexabydroxycyclohexane), ON 2344 (DRF 2593); Dexlipotam, Chemical Name: 5(R)-(1,2-Dithiolan-3-yl) pentaloic 35 acid; HQL 975, Chemical Name: 3-[4- [2-(5-Methyl-2-phenyloxazol-4-yl) ethoxy]phenyl]-2(S)-(propylamino) propionic acid; YM 268, Chemical Name: 5,5'-Methylene-bis(1,4-phenylene)bismethylenebis (thiazolidine-2,4-dione). I PPAR agonists under development include: Reglitazar (JTT 501, PNU 182716, PNU 716) (Chemical Name: Isoxazolidien-3, 5-dione, i 4-[[4-(2-phenyl-5-methyl)-1,3-oxazolyl]ethoxyphenyl-4] methyl-, (4RS)); I(RP 297, Chemical Name: 10 5-(2,4-DioXothiazolidin-5-ylmethyl)-2-methoxy-N-[4-(trifluoromethyl) benzylbenzamide; R 119702 (CI 1037, CS 011) ChemicalName: (/-)-5-[4-(5-Methoxy- 1H benzimidazol-2-ylmethoxy)benzyl] thiazolin-2,4-dione; hydrochloride; 15 DRF 2189, Chemical Name: 5-[[4-[2-(1-Indolyl)ethoxy]phenyl]methyl] thiazolidine-2,4-dione; cortisol synthesis inhibitors such as Ketoconazole, econazole or miconazole; growth hormones such as somatropin or somatonorm and their derivatives such as human growth hormone fusion proteins such as ALBUTROPIN; polyethylene glycol growth hormones such as the cysteine-pegylated growth hormone, BT 005 (Bolder BioTechnology Inc.); growth hormone secretagogues such as, for example, SM 130686 (Sumitomo) capromorelin (Pfizer), mecasermin (Fujisawa), sermorelin (Salk Institute, Bio-Technology General), somatrem, somatomedin (C Llorente; Pharmacia Corporation) examorelin, tabimorelin; CP 464709 (Pfizer), LY 426410 and LY 444711 (Lilly); 8-(aminoalkoxyimino)-8H-dibenzo[a,e]triazolo[4,5-c]cycloheptenes as disclosed in WO2002057241, 2-substituted dibenzo[a,e]1,2,3-triazolo[4,5-c][7]annulen-8-ones as described in WO2002056873 growth hormone releasing peptides GHRP-6 and GHRP-1 as described in U.S. Patent No. 4,411,890, and publications WO 89/07110, WO 89/07111, B-HT920, hexarelin and GHRP-2 as described in WO 93/04081 or growth hormone releasing hormone (GHRH, also designated GRF) and its analogs, somatomedins including IGF-1 and IGF-2 and their derivatives such as SomatoKine - a recombinant fusion of insulin-like growth factor-1 and its binding protein, BP-3, alpha-2-adrenergic agonists such as clonidine, xylazine, detomidine and medetomidine or serotonin 5HTID agonists such as surnitriptan or agents which inhibit somatostatin or its release such as physostigmine and pyridostigmine, ThGRF 1-44 (Theratechnologies); L 165166 (Merck & Company); dipeptide derivatives as described in W09858947, Inhibitors of dipeptidyl peptidase IV such as amino-acylpyrrolidine nitrile as described in US6521644, WO95/15309 and WO98/19998; Beta-amino heterocyclic dipeptidyl peptidase inhibitors such as those described in US20030100563 and W02003082817; growth hormone releasing compounds as described in US20030055261, US20030040483, EP 18 072, EP 83 864, WO 89/07110, WO 89/01711, WO 89/10933, WO 88/9780, WO 83/02272, WO 91/18016, WO 92/01711, WO 93/04081, WO 9514666, EP0923539, U.S. Patent Nos. 5,206,235, 5,283,241, 5,284,841, 5,310,737, 5,317,017, 5,374,721, 5,430,144, 5,434,261, 5,438,136, 5,494,919, 5,494,920, 5,492,916, 5,536,716 and 5,578,593, WO 94/13696, WO 94/19367, WO 95/03289, WO 95/03290, WO 95/09633, WO 95/11029, WO 95/12598, WO 95/13069, WO 95/14666, WO 95/16675, WO 95/16692, WO 95/17422,

WO 95/17423, WO 95/34311, and WO 96/02530, Piperidines, pyrrolidines and hexahydro-1H-azepines as described in US5804578, US5783582, WO2004007468, AMIDO SPIROPIPERIDINES such as those described in WO0104119, 2-amino-5-pyrimidine acetic acid compounds including 2-[(5,6-Dimetlhyl-2-benzoimidazolyl)amino]-4-hydroxy-6-methyl-5-pyrimidine acetic acid (2) and 2-[(5,6-Dimethyl-2-benzoimidadazolyl)amino]-4-hydroxy-6-methyl-5- pyrimidine acetic acid, ethyl ester as described in US6329383, benzimidazoles as described in EP1155014, analogous peptidyl compounds related to GRF and the peptides of U.S. Patent 4,411,890, antagonists of gonadotropin releasing hormone such as those described in W00170228, WO0170227, W00170228, W00069433, WO0004013, W0995156, W09951595,W09951231-4, W09941251-2, WO9921557, W09921553 and 6-AZAINDOLE COMPOUNDS as described in W00053602, W00053185, W00053181, WO0053180, W00053179, W00053178, US6288078; IGF-1 secretagogues; insulin-like growth factor-2 (IGF- 2 or somatomedin A) and IGF-2 secretagogues; myostatin antagonists and compounds which inhibit fibroblast growth factor receptor-3 (FGFR-3) tyrosine kinase.

The polymeric substances included are also preferably water-soluble at room temperature. A non-limiting list of such polymers include poly(alkylene oxide) homopolymers such as poly(ethylene glycol) or poly(propylene glycols), poly(oxyethylenated polyols), copolymers thereof and block copolymers thereof, provided that the water solubility of the block copolymers is maintained.

As an alternative to PEG-based polymers, effectively non-antigenic materials such as dextran, poly(vinyl pyrrolidones), poly(acrylamides), poly(vinyl alcohols), carbohydrate-based polymers, and the like can be used. Indeed, the activation of α- and ε-terminal groups of these polymeric substances can be effected in fashions similar to that used to convert poly(alkylene oxides) and thus will be apparent to those of ordinary skill. Those of ordinary skill in the art will realize that the foregoing list is merely illustrative and that all polymer materials having the qualities described herein are contemplated. For purposes of the present invention, "effectively non-antigenic" means all materials understood in the art as being nontoxic and not eliciting an appreciable immunogenic response in mammals.

***Definitions***

The following is a list of abbreviations and the corresponding meanings as used interchangeably herein:

| | |
|---|---|
| g | gram(s) |
| mg | milligram(s) |
| ml or mL | milliliter(s) |
| RT | room temperature |
| PEG | poly (ethylene glycol) |

The complete content of all publications, patents, and patent applications cited in this disclosure are herein incorporated by reference as if each individual publication, patent, or patent application were specifically and individually indicated to be incorporated by reference.

Although the foregoing invention has been described in some detail by way of illustration and example for the purposes of clarity of understanding, it will be readily apparent to one skilled in the art in light of the teachings of this invention that changes and modifications can be made without departing from the spirit and scope of the present invention. The following examples are provided for exemplification purposes only and are not intended to limit the scope of the invention, which has been described in broad terms above.

In the following examples, the hGH is that of SEQ ID NO:1. It is understood that other members of the hGH or agonist variant thereof family of polypeptides could also be pegylated in a similar manner as exemplified in the subsequent examples.

<u>EXAMPLES</u>

Example 1

Branched 40,000 MW PEG-butyrylaldhyde hGH

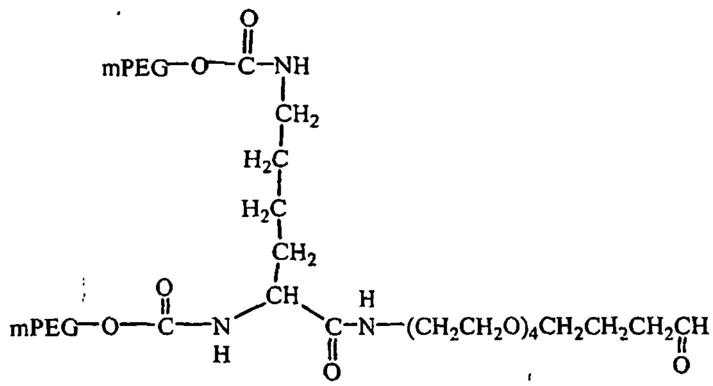

This example demonstrates a method for generation of substantially homogeneous preparations of N-terminally monopegylated hGH by reductive alkylation. Methoxy-branched PEG-butyrylaldehyde reagent of approximately 40,000 MW (Shearwater Corp.) was selectively coupled via reductive amination to the N-terminus of hGH by taking advantage of the difference in the relative $pK_a$ value of the primary amine at the N-terminus versus $pK_a$ values of primary amines at the ε-amino position of lysine residues. hGH protein dissolved at 10 mg/mL in 25 mM Hepes (Sigma Chemical, St. Louis, MO) pH 7.0, (optionally 25 mM MES (Sigma Chemical , St. Louis, MO) pH 6.0, 10 mM Sodium Acetate (Sigma Chemical , St. Louis, MO) pH 4.5), was reacted with Methoxy-PEG- butyrylaldehyde, M-PEG-ALD, (Shearwater Corp., Huntsville, AL) by addition of M-PEG-ALD to yield a relative PEG:hGH molar ratio of 2:1. Reactions were catalyzed by addition of stock 1M $NaCNBH_4$ (Sigma Chemical , St. Louis, MO), dissolved in $H_20$, to a final concentration of 10-50 mM. Reactions were carried out in the dark at RT for 18-24 hours. Reactions were stopped by addition of 1 M Tris (Sigma Chemical, St. Louis, MO) ~pH 7.6 to a final Tris concentration of 50 mM or diluted into appropriate buffer for immediate purification.

Table 1 shows the percent, as determined by Size Exclusion Chromatography, of multi-PEGylated species, mono-PEGylated conjugate, un-reacted PEG, and final purification yield for 40K branched PEG-aldehyde and 40K branched PEG-butyrylaldehyde. The PEG-butyrylalehyde results in increased mono-PEGylated conjugate, decreased levels of un-reacted PEG, and increased final yield compared to PEG-aldehyde.

TABLE 1

| Comparison of 40K Branched PEG-ALD-hGH and 40K branched PEG-Butyrylaldehyde-hGH | | |
|---|---|---|
| Species in the reaction mix: | | |
| | 40K PEG-aldehyde hGH | 40K PEG-butyrylaldehyde-hGH |
| | | |
| multi-PEG product | 4.02% | 5.03% |
| mono-PEG product | 48.70% | 61.02% |
| un-reacted hGH | 41.80% | 29.20% |
| | | |
| Final purification yield | 30.80% | 44.70% . |

Example 2

Straight Chain 30,000 MW PEG-butyrylaldehyde hGH

Methoxy-linear 30,000 MW PEG-butyrylaldehyde reagent is coupled to the N-terminus of hGH using the procedure described for Example 1.

Example 3

Straight chain 20,000 MW PEG-butyrylaldehyde hGH

Methoxy-linear 20,000 MW PEG-butyrylaldehyde reagent is coupled to the N-terminus of hGH using the procedure described for Example 1.

Example 4

Purification of Pegylated hGH

Pegylated hGH species were purified from the reaction mixture to >95% (SEC analysis) using a single ion exchange chromatography step.

*Anion exchange chromatography*

The PEG hGH species were purified from the reaction mixture to >95% (SEC analysis) using a single anion exchange chromatography step. Mono-pegylated hGH was purified from unmodified hGH and multi-pegylated hGH species using anion exchange chromatography. A typical 20K butyrylaldehyde hGH reaction mixture (5-100 mg protein), as described above, was purified on a Q-Sepharose Hitrap column (1 or 5 mL)(Amersham Pharmacia Biotech, Piscataway, NJ) or Q-Sepharose fast flow column (26/20, 70 mL bed volume)(Amersham Pharmacia Biotech, Piscataway, NJ) equilibrated in 25 mM HEPES, pH 7.3 (Buffer A). The reaction mixture was diluted 5-10X with buffer A and loaded onto the column at a flow rate of 2.5 mL/min. The column was washed with 8 column volumes of buffer A. Subsequently, the various hGH species were eluted from the column in 80-100 column volumes of Buffer A and a linear NaCl gradient of 0-100 mM. The eluant was monitored by absorbance at 280 nm ($A_{280}$) and 5 mL fractions were collected. Fractions were pooled as to extent of pegylation, e.g., mono, di, tri etc. (as assessed in example 15). The pool was then concentrated to 0.5-5 mg/mL in a Centriprep YM10 concentrator (Amicon, Technology Corporation, Northborough, MA). Protein concentration of pool was determined by $A_{280}$ using an extinction coefficient of 0.78

*Cation Exchange Chromatography*

Cation exchange chromatography is carried out on an SP Sepharose high performance column (Pharmacia XK 26/20, 70 ml bed volume) equilibrated in 10 mM sodium acetate pH 4.0 (Buffer B). The reaction mixture is diluted 10X with buffer B and loaded onto the column at a flow rate of 5 mL/min. Next the column is washed with 5 column volumes of buffer B, followed by 5 column volumes of 12% buffer C (10 mM acetate pH 4.5, 1 M NaCl). Subsequently, the PEG-hGH species are eluted from the column with a linear gradient of 12 to 27% buffer C in 20 column volumes. The eluant is monitored at 280 nm and 10 mL fractions were collected. Fractions are pooled according to extent of pegylation (mono, di, tri etc.), exchanged into 10 mM acetate pH 4.5 buffer and concentrated to 1-5 mg/mL in a stirred cell fitted with an Amicon YM10 membrane. Protein concentration of pool is determined by A280 nm using an extinction coefficient of 0.78.

Example 5

Biochemical Characterization

The purified pegylated hGH pools were characterized by non-reducing SDS-PAGE, non-denaturing Size Exclusion Chromatography, and peptide mapping.

*Size Exclusion High Performance Liquid Chromatography (SEC-HPLC)*

*Non-denaturing SEC-HPLC*

The reaction of Methoxy-PEG of various attachment chemistries, sizes, linkers, and geometries with hGH, anion exchange purification pools and final purified products were assessed using non-denaturing SEC-HPLC. Analytical non-denaturing SEC-HPLC was carried out using a column, Superdex 200 7.8 mm X 30 cm, (Amersham Bioscience, Piscataway, NJ) in 20 mM Phosphate pH 7.2, 150 mM NaCl at a flow rate of 0.5 mL/minute (optionally Tosohaas G4000PWXL Amersham Bioscience, Piscataway, NJ). PEGylation greatly increases the hydrodynamic volume of the protein resulting in a shift to an earlier retention time. New species were observed in the PEG aldehyde hGH reaction

mixtures along with unmodified hGH. These PEGylated and non-PEGylated species were separated on Q-Sepharose chromatography, and the resultant purified mono PEG-Aldehyde hGH species were subsequently shown to elute as a single peak on non-denaturing SEC (> 95% purity). The Q-Sepharose chromatography step effectively removed free PEG, hGH, and multi PEGylated hGH species from the mono-Pegylated hGH

*Denaturing SEC-HPLC*

The reaction of the butyrylaldehyde polyethylene glycols with hGH, anion exchange purification, and final purified products are assessed using denaturing SEC-HPLC. Analytical denaturing SEC-HPLC is carried out using a Tosohaas 3000SWXL column 7.8 mm X 30 cm (Tosohaas Pharmacia Biotech, Piscataway, NJ) in 100 mM Phosphate pH 6.8, 0.1% SDS at a flow rate of 0.8 mL/minute. PEGylation greatly increases the hydrodynamic volume of the protein resulting in a shift to an earlier retention time. PEGylated and non-PEGylated species are separated on Q-Sepharose chromatography

*SDS PAGE/PVDF transfer*

SDS-PAGE was used to assess the reaction PEG butyrylaldehyde with hGH and the purified final products. SDS-PAGE was carried out on 1 mm thick 10-20% Tris tricine gels (Invitrogen, Carlsbad, CA) under reducing and non-reducing conditions and stained using a Novex Colloidal Coomassie™ G-250 staining kit (Invitrogen, Carlsbad, CA). Bands are blotted onto PVDF membrane for subsequent N-terminal sequence identification.

*Analytical anion exchange HPLC*

The PEG butyrylaldehyde/hGH reaction mixture, anion exchange purification fractions, and final purified products were assessed using analytical anion exchange HPLC. Analytical anion exchange HPLC was carried out using a Tosohaas Q5PW or DEAE-PW anion exchange column, 7.5 mm x 75 mm (Tosohaas Pharmacia Biotech, Piscataway, NJ) in 50 mM Tris ph 8.6 at a flow rate of 1 mL/min. Samples were eluted with a linear gradient of 5-200 mM NaCl.

*N-terminal Sequence and Peptide Mapping*

Automated Edman degradation chemistry was used to determine the $NH_2$-terminal protein sequence. An Applied Biosystems Model 494 Procise sequencer (Perkin Elmer, Wellesley, MA) was employed for the degradation. The respective PTH-AA derivatives were identified by RP-HPLC analysis in an on-line fashion employing an Applied Biosystems Model 140C PTH analyzer fitted with a Perkin Elmer/Brownlee 2.1 mm i.d. PTH-C18 column.

Tryptic digest were performed at a concentration of 1 mg/mL and typically 50 ug of material is used per digest. Trypsin was added such that the trypsin to PEG-hGH ratio was 1:30 (w/w). Tris buffer was present at 30 mM, pH 7.5. Samples were incubated at room temperature for 16 $\pm$ 0.5 hours. Reactions were quenched by the addition of 50 $\mu$L of 1N HCl per mL of digestion solution. Samples were diluted, prior to placing the samples in the autosampler, to a final concentration of 0.25 mg/ml in 6.25 % acetonitrile. Acetonitrile was added first (to 19.8% acetonitrile), mixed gently, and then water is added to final volume (four times the starting volume). Extra digestion solution may be removed and stored for up to 1 week at -20°C.

A Waters Alliance 2695 HPLC system was used for analysis, but other systems should produce similar results. The column used was an Astec C-4 polymeric 25 cm x 4.6 mm column with 5 $\mu$m particles. Experiments were conducted at ambient temperature on a typical load of 50 $\mu$g of protein per sample. Buffer A is 0.1% trifluoroacetic acid in water; buffer B is 0.085% trifluoroacetic acid in acetonitrile. The gradient was as follows:

| Time | A% | B% | C% | D% | Flow | Curve |
|---|---|---|---|---|---|---|
| 0.00 | 0.0 | 0.0 | 100.0 | 0.0 | 1.000 | 1 |
| 90.00 | 0.0 | 0.0 | 55.0 | 45.0 | 1.000 | 6 |
| 90.10 | 0.0 | 0.0 | 0.0 | 100.0 | 1.000 | 6 |
| 91.00 | 0.0 | 0.0 | 0.0 | 100.0 | 1.000 | 6 |
| 91.10 | 0.0 | 0.0 | 100.0 | 0.0 | 1.000 | 6 |
| 95.00 | 0.0 | 0.0 | 100.0 | 0.0 | 1.000 | 6 |

The column is heated to 40°C using a heat jacket. Peaks were detected using a Waters 996 PDA detector collecting data between 210 and 300 nm. The extracted chromatogram at 214 nm was used for sample analysis.

Tryptic maps were performed for hGH, 40K branched PEG-aldehyde, and 40K branched butyrylaldehyde (Figure 1). The N-terminal tryptic fragment was referred to as T-1. The percent T-1 present compared to unPEGylated hGH is shown in Table 2. This data suggest that 90% of the PEG modification is at the N-terminus with remainder apparently linked to one of several possible lysine residues using PEG-aldehyde compared to greater than 98% at the N-terminus using PEG-butyrylaldehyde.

TABLE 2

| | % T-1 present | % T-1 present compared to unPEGylated hGH |
|---|---|---|
| hGH | 28.0% | |
| PEG-aldehyde/hGH | 2.6% | 9.2% |
| PEG-butyrylaldehyde/hGH | 0.3% | 1.2% |

Example 6

*Pharmacodynamic Studies*

*Rat weight Gain*

Female Sprague Dawley rats, hypophysectomized at Taconic Labs, were prescreened for growth rate for a period of 7 to 11 days. Rats were divided into groups of eight. Group 1 consisting of rats given either daily or day 0 and day 6 subcutaneous dose of vehicle. Group 2 were given daily subcutaneous dose of GH (30μg/rat/dose). Group 3 were given subcutaneous doses of GH on day 0 and day 6 (180μg/rat/dose). Group 4 were given subcutaneous doses of 40k branched PEG-butyrylaldehyde-hGH on day 0,6 (180μg/rat/dose). Hypophysectomized rats were monitored for weight gain by weighing at least every other day during the study. Figure 3 & 4.

*Rat tibia length*

Animals in 11 Day weight gain studies at day 11 were sacrificed, left tibias were removed and X-rayed and bone lengths were measured using a caliper. Figure 5

*IGF-1 Studies*

Animals from six-day weight gain studies were used. Blood samples were taken at the various times during the study and the serum IGF-1 levels determined by ELISA. Figure 6

*Serum biochemistry studies*

Animals in 11 Day weight gain studies were used to evaluate serum biochemistry values on Day 7 following cumulative administration of 1.8 mg/kg on Day 0 and 6 as shown in Table 3.

**Table 3**

| Assay | Vehicle | hGH (11 days at 300 μg/kg/day) | PEG-hGH (1.8 mg/kg at Day 0 and 6) |
|---|---|---|---|
| ALB (g/dL) | 4.07±0.04 | 4.06±0.05 | 4.18±0.03*† |
| ALP (U/L) | 311±15 | 309±16 | 280±14 |
| ALT (U/L) | 53.6±2.4 | 51.1±2.2 | 51.3±2.6 |
| AST (U/L) | 149±7 | 131±4 | 137±11 |
| BLIN (mg/dL) | 37.4±1.6 | 26.7±1.5* | 27.9±0.6* |
| $Ca^{2+}$ (mg/dL) | 10.9±0.1 | 11.5±0.1* | 11.0±0.1 † |
| Chol (mg/dL) | 85.1±2.8 | 76.9±3.6* | 115.3±2.8*† |
| CRE (mg/dL) | 0.62±0.02 | 0.60±0.01 | 0.59±0.01 |
| Glucose (mg/dL) | 73.4±4.7 | 79.6±4.7 | 67.1±8.9 |
| Phos (mg/dL) | 8.26±0.28 | 9.59±0.16* | 8.42±0.23† |

(continued)

| Assay | Vehicle | hGH (11 days at 300 μg/kg/day) | PEG-hGH (1.8 mg/kg at Day 0 and 6) |
|---|---|---|---|
| TP (g/dL) | 6.36±0.10 | 6.48±0.10 | 6.39±0.06 |
| TBA (μmol/L) | 10.0±0.5 | 7.6+0.4* | 11.0±0.4† |
| SDH (U/L) | 11.9±1.0 | 9.7±1.4 | 10.6±0.7 |
| Triglycerides (mg/dL) | 57.4±4.0 | 47.8±5.7 | 45.7±3.9* |
| LDH (U/L) | 786±72 | 762±94 | 914±189 |
| $Na^+$ (mmol/L) | 147±0.5 | 146±0.8 | 145±0.6* |
| $K^+$ (mmol/L) | 5.94±0.09 | 6.31±0.16* | 6.71±0.17* † |
| $Cl^-$ (mmol/L) | 103±0.5 | 102±0.5 | 102±0.6 |

* p <0.10 vs Vehicle, † p <0.05 vs hGH.
ALB, albumin; ALP, Alkaline Phosphatase; ALT, alanine aminotransferase; AST, aspartate aminotransferase; BUN, blood urea nitrogen; $Ca^{2+}$, calcium; Chol, cholesterol; CRE, creatinine; Phos, phosphate; TP, total protein; TBA, total bile acid; SDH, sorbitol dehydrogenase; LDH, lactic dehydrogenase; $Na^+$, sodium; $K^+$, potassium; $Cl^-$, chlorine.

Blood urea nitrogen concentration at Day 11 decreased significantly (p <0.10) by the same extent relative to the vehicle group in the hGH- and PHA-794428-treated animals. This is indicative of increased nitrogen utilization as a result of new protein synthesis during enhanced growth.

*Pharmacokinetic Studies*

Pharmacokinetic studies were conducted in normal, cannulated Sprague-Dawley male rats. Injections were made as a single subcutaneous bolus of 100μg/kg/rat GH or PEG-GH using six rats per group. Blood samples were taken over one to five days as appropriate for assessment of relevant PK parameters. GH and PEG-GH blood levels were monitored at each sampling using immuno-assay.

*hGH Immunoassay*

hGH and pegylated hGH protein concentration levels in mouse and cynomolgus monkey plasma were determined using the hGH AutoDELFIA kit fluorescence immunoassay (PerkinElmer). Rat and human IGF-1 levels were monitored by immunoassay kit (Diagnostic System Laboratories)

*Non-compartmental Pharmacokinetic Properties for hGH-PEG conjugate of Example 1 in Non-human Primates.*

The hGH-PEG conjugate of Example 1 was administered to cynomolgus monkeys as 0.18 mg/kg intravenous (iv) or subcutaneous (sc) bolus injections (Table 4). PK parameters were determined using mean data for n=3 animals. Plasma concentrations were measured using the AutoDELFIA kit fluorescence immunoassay (PerkinElmer) and a standard curve pre-determined for the PEG-GH conjugate.

Table 4

| Dose (mg/kg) | iv 0.18 / sc 0.18 |
|---|---|
| CL, iv (ml/hr/kg) | 0.8 |
| Vss (ml/kg) | 28.0 |
| T1/2, iv (hr) | 25.0 |
| T1/2, sc (hr) | 61.2 |
| SC AUC (μg/ml*hr) | 195 |
| SC Bioavailability (%) | 84 |
| Tmax, sc (hr) | 32 |

## Claims

1. Use of a poly(ethylene glycol)-modified hGH having the structure of formula I or II,

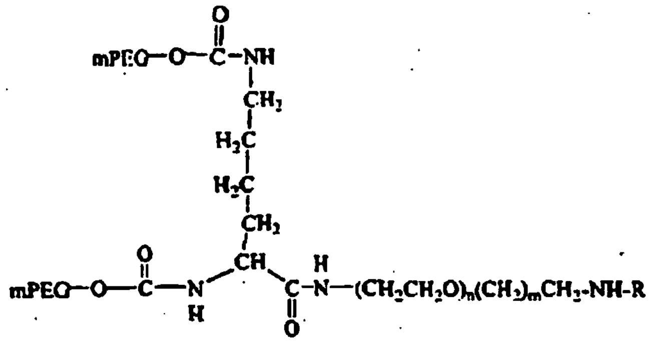

Formula I

or

$$mPEG\text{-}O(CH_2CH_2O)_n(CH_2)_mCH_2\text{-}NH\text{-}R \qquad \text{Formula II}$$

wherein
n is an integer between 1 and 10;
m is an integer between 1 and 10;
R is human growth hormone or methionyl growth hormone,
alone or in combination with another therapeutic agent in the manufacture of a medicament for the prevention and/or treatment of a disease or disorder in which use of growth hormone is beneficial, wherein said disease or disorder is selected from the group consisting of Traumatic brain injury, Subarachnoid haemorrhage, Noonan's syndrome, Idiopathic short stature (ISS), Very low birth weight (VLBW), Short stature due to glucocorticoid treatment in children, and Failure of growth catching for short premature children.

2. The use according to claim 1, wherein said disease or disorder in which use of GH is beneficial is ISS.

3. The use according to claim 1 or claim 2, wherein n equals 4 and m equals 3.

4. The use according to claim 3, wherein said poly (ethylene glycol)-modified hGH is having the structure of formula I with n equals 4 and m equals 3.

5. The use according to any one of claims 1 to 4, wherein said human growth hormone comprises the amino acid sequence of SEQ ID NO:1.

6. The use according to claim 5, wherein greater than 90% of said polyethylene glycol is conjugated to an amino-terminal phenylalanine of the amino acid sequence of SEQ ID NO:1.

7. The use according to claim 6, wherein greater than 95% of said polyethylene glycol is conjugated to an amino-terminal phenylalanine of the amino acid sequence of SEQ ID NO:1.

8. The use according to any one of claims 1 to 7, wherein each mPEG has a molecular weight of about 20 kDa.

9. A composition comprising the human growth hormone-PEG conjugate of formula I or II in combination with another therapeutic agent, and at least one pharmaceutically acceptable carrier.

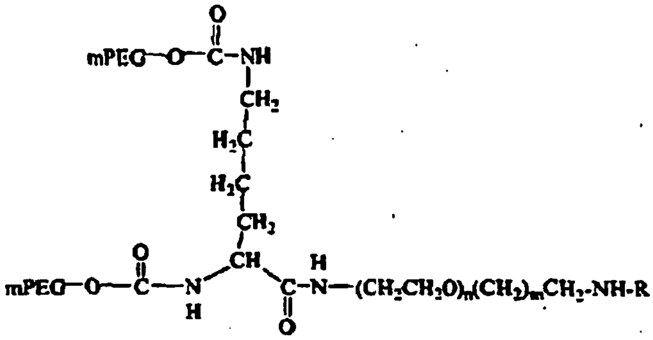

**Formula I**

or

mPEG-O($CH_2CH_2O$)$_n$($CH_2$)$_m$$CH_2$-NH-R Formula II

wherein
n is an integer between 1 and 10;
m is an integer between 1 and 10;
R is human growth hormone or methionyl growth hormone.

**10.** The composition of claim 9, wherein said poly(ethylene glycol) -modified hGH is having the structure of formula I with n equals 4 and m equals 3.

**Patentansprüche**

**1.** Verwendung eines Poly(ethylenglykol)-modifizierten hGH mit der Struktur der Formel I oder II,

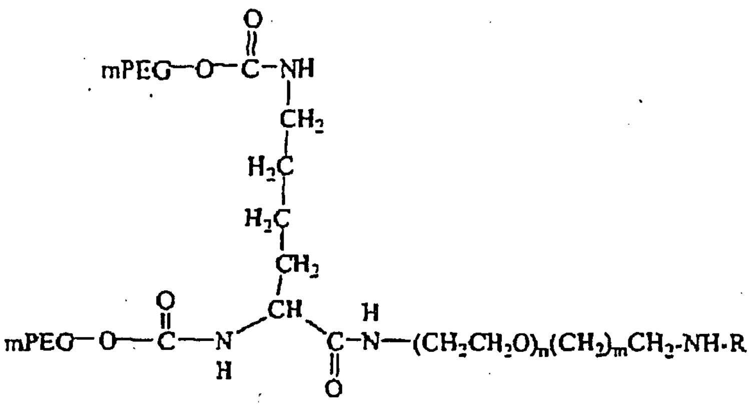

Formel I

oder

mPEG-O($CH_2CH_2O$)$_n$($CH_2$)$_m$$CH_2$-NH-R Formel II

worin n eine ganze Zahl zwischen 1 und 10 ist;
m eine ganze Zahl zwischen 1 und 10 ist;
R ein humanes Wachstumshormon oder Methionyl-Wachstumshormon ist,
allein oder in Kombination mit einem anderen therapeutischen Mittel in der Herstellung eines Medikamentes zur Prävention und/oder Behandlung einer Erkrankung oder Störung, bei der die Verwendung eines Wachstumshormons

vorteilhaft ist, wobei die Erkrankung oder Störung ausgewählt ist aus der Gruppe, bestehend aus traumatischer Hirnverletzung, Subarachnoidalblutung, Noonan-Syndrom, idiopathischem Kleinwuchs (ISS), sehr geringem Geburtsgewicht (VLBW), Kleinwuchs bei Kindern aufgrund von Glucocorticoidbehandlung und Wachstumsrückstand, der kleine Frühgeborene erfasst.

**2.** Verwendung gemäß Anspruch 1, wobei die Erkrankung oder Störung, bei der die Verwendung von GH vorteilhaft ist, ISS ist.

**3.** Verwendung gemäß Anspruch 1 oder Anspruch 2, wobei n gleich 4 und m gleich 3 ist.

**4.** Verwendung gemäß Anspruch 3, wobei das Poly(ethylenglykol)-modifizierte hGH die Struktur der Formel I hat, wobei n gleich 4 ist und m gleich 3 ist.

**5.** Verwendung gemäß einem der Ansprüche 1 bis 4, wobei das humane Wachstumshormon die Aminosäuresequenz SEQ ID NO: 1 umfasst.

**6.** Verwendung gemäß Anspruch 5, wobei mehr als 90% des Polyethylenglykols mit einem aminoterminalen Phenylalanin der Aminosäuresequenz SEQ ID NO: 1 konjugiert sind.

**7.** Verwendung gemäß Anspruch 6, wobei mehr als 95% des Polyethylenglykols mit einem aminoterminalen Phenylalanin der Aminosäuresequenz SEQ ID NO: 1 konjugiert sind.

**8.** Verwendung gemäß einem der Ansprüche 1 bis 7, wobei jedes mPEG ein Molekulargewicht von etwa 20 kDa hat.

**9.** Zusammensetzung, umfassend das humanes Wachstumshormon-PEG-Konjugat der Formel I oder II in Kombination mit einem anderen therapeutischen Mittel und mindestens einen pharmazeutisch annehmbaren Träger:

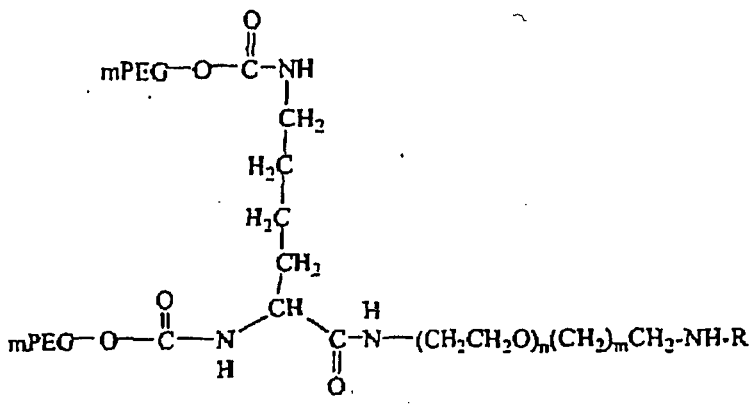

Formel I

oder

$mPEG-O(CH_2CH_2O)_n(CH_2)_mCH_2-NH-R$ Formel II

worin n eine ganze Zahl zwischen 1 und 10 ist;
m eine ganze Zahl zwischen 1 und 10 ist;
R humanes Wachstumshormon oder Methionyl-Wachstumshormon ist.

**10.** Zusammensetzung nach Anspruch 9, wobei das Poly(ethylenglykol)-modifizierte hGH die Struktur der Formel I hat, wobei n gleich 4 ist und m gleich 3 ist.

## Revendications

**1.** Utilisation d'une hGH modifiée par du poly(éthylène glycol), ayant la structure de la formule I ou II,

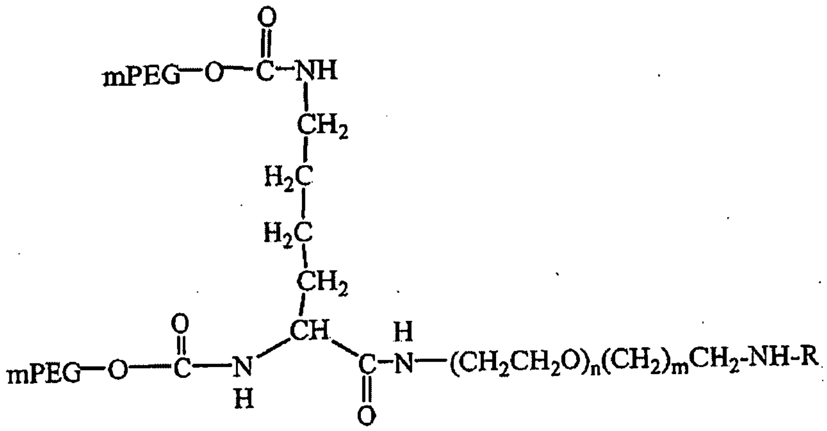

**Formule I**

ou

$$mPEG\text{-}O(CH_2CH_2O)_n(CH_2)_mCH_2\text{-}NH\text{-}R \qquad \textbf{Formule II}$$

formules dans lesquelles:

n est un entier compris entre 1 et 10 ;
m est un entier compris entre 1 et 10 ;
R est l'hormone de croissance humaine ou la méthionyl-hormone de croissance,
seule ou en combinaison avec un autre agent thérapeutique,

dans la fabrication d'un médicament pour la prévention et/ou le traitement d'une maladie ou d'un trouble dans laquelle/lequel l'utilisation d'hormone de croissance est bénéfique, ladite maladie ou ledit trouble étant sélectionné(e) dans le groupe consistant en le traumatisme crânien, l'hémorragie subarachnoïde, le syndrome de Noonan, la petite taille idiopathique (ISS), le très faible poids à la naissance (VLBW), la petite taille due à un traitement glucocorticoïde chez les enfants, et le retard de croissance chez les prématurés de petite taille.

**2.** Utilisation selon la revendication 1, dans laquelle ladite maladie ou ledit trouble dans laquelle/lequel l'utilisation de GH est bénéfique est l'ISS.

**3.** Utilisation selon la revendication 1 ou la revendication 2, dans laquelle n est égal à 4 et m est égal à 3.

**4.** Utilisation selon la revendication 3, dans laquelle ladite hGH modifiée par du poly(éthylène glycol) a la structure de formule I, avec n égal 4 et m égal 3.

**5.** Utilisation selon l'une quelconque des revendications 1 à 4, dans laquelle ladite hormone de croissance humaine comprend la séquence d'acides aminés SEQ ID N°1.

**6.** Utilisation selon la revendication 5, dans laquelle plus de 90 % dudit polyéthylène glycol est conjugué avec une phénylalanine amino-terminale de la séquence d'acides aminés SEQ ID N°1.

**7.** Utilisation selon la revendication 6, dans laquelle plus de 95 % dudit polyéthylène glycol est conjugué avec une phénylalanine amino-terminale de la séquence d'acides aminés SEQ ID N°1.

**8.** Utilisation selon l'une quelconque des revendications 1 à 7, dans laquelle chaque mPEG a un poids moléculaire d'environ 20 kDa.

**9.** Composition comprenant le conjugué hormone de croissance humaine-PEG de formule I ou II en combinaison avec un autre agent thérapeutique et au moins un support pharmaceutiquement acceptable :

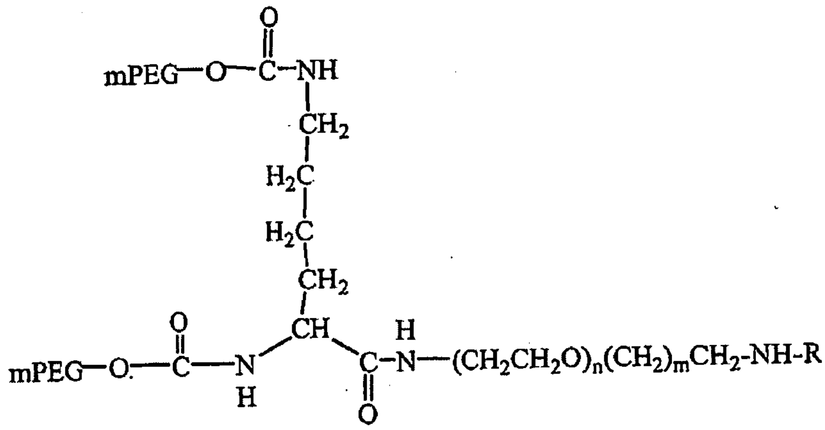

**Formule I**

ou

**mPEG-O$(CH_2CH_2O)_n(CH_2)_mCH_2$-NH-R** **Formule II**

formules dans lesquelles:

n est un entier compris entre 1 et 10 ;
m est un entier compris entre 1 et 10 ;
R est l'hormone de croissance humaine ou la méthionyl-hormone de croissance,

**10.** Composition selon la revendication 9, dans laquelle ladite hGH modifiée par du poly(éthylène glycol) a la structure de formule I, avec n égal 4 et m égal 3.

FIG. 1

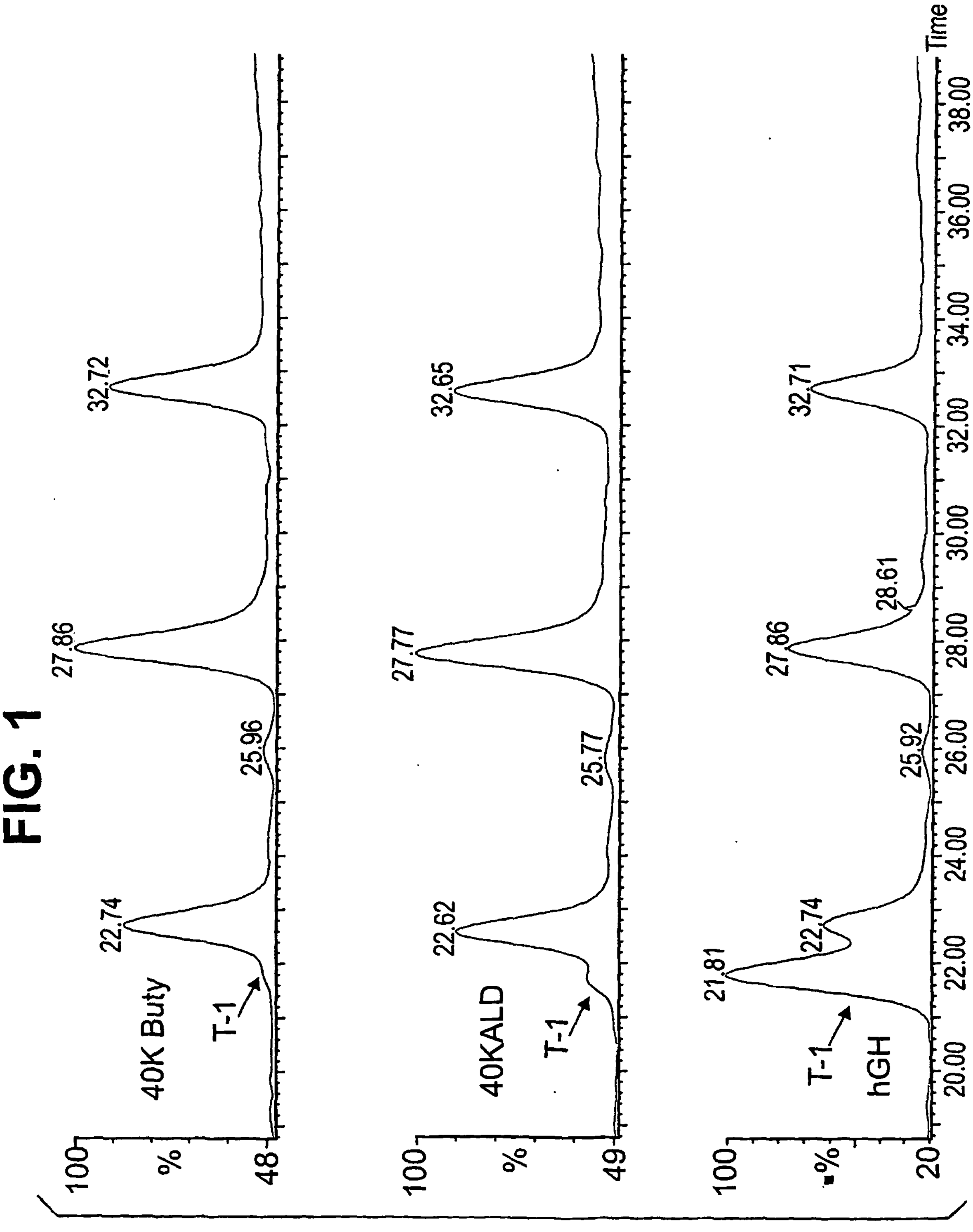
40K Buty
T-1
22.74
25.96
27.86
32.72
100
%
48
40KALD
T-1
22.62
25.77
27.77
32.65
100
%
49
T-1
hGH
21.81
22.74
25.92
27.86
28.61
32.71
100
%
20
20.00
22.00
24.00
26.00
28.00
30.00
32.00
34.00
36.00
38.00
Time

# FIG. 2

| | | | | | | | | | | | | |
|---|---|---|---|---|---|---|---|---|---|---|---|---|
| Phe | Pro | Thr | Ile | Pro | Leu | Ser | Arg | Leu | Phe | Asp | Asn | 12 |
| Ala | Met | Leu | Arg | Ala | His | Arg | Leu | His | Gln | Leu | Ala | 24 |
| Phe | Asp | Thr | Tyr | Gln | Glu | Phe | Glu | Glu | Ala | Tyr | Ile | 36 |
| Pro | Lys | Glu | Gln | Lys | Tyr | Ser | Phe | Leu | Gln | Asn | Pro | 48 |
| Gln | Thr | Ser | Leu | Cys | Phe | Ser | Glu | Ser | Ile | Pro | Thr | 60 |
| Pro | Ser | Asn | Arg | Glu | Glu | Thr | Gln | Gln | Lys | Ser | Asn | 72 |
| Leu | Glu | Leu | Leu | Arg | Ile | Ser | Leu | Leu | Leu | Ile | Gln | 84 |
| Ser | Trp | Leu | Glu | Pro | Val | Gln | Ser | Leu | Arg | Ser | Val | 96 |
| Phe | Ala | Asn | Ser | Leu | Val | Tyr | Gly | Ala | Ser | Asp | Ser | 108 |
| Asn | Val | Tyr | Asp | Leu | Leu | Lys | Asp | Leu | Glu | Glu | Gly | 120 |
| Ile | Gln | Thr | Leu | Met | Gly | Arg | Leu | Glu | Asp | Gly | Ser | 132 |
| Pro | Arg | Thr | Gly | Gln | Ile | Phe | Lys | Gln | Thr | Tyr | Ser | 144 |
| Lys | Phe | Asp | Thr | Asn | Ser | His | Asn | Asp | Asp | Ala | Leu | 156 |
| Leu | Lys | Asn | Tyr | Gly | Leu | Leu | Tyr | Cys | Phe | Arg | Lys | 168 |
| Asp | Met | Asp | Lys | Val | Glu | Thr | Phe | Leu | Arg | Ile | Val | 180 |
| Gln | Cys | Arg | Ser | Val | Glu | Gly | Ser | Cys | Gly | Phe | | 191 |

SEQ. ID NO.: 1

FIG. 3

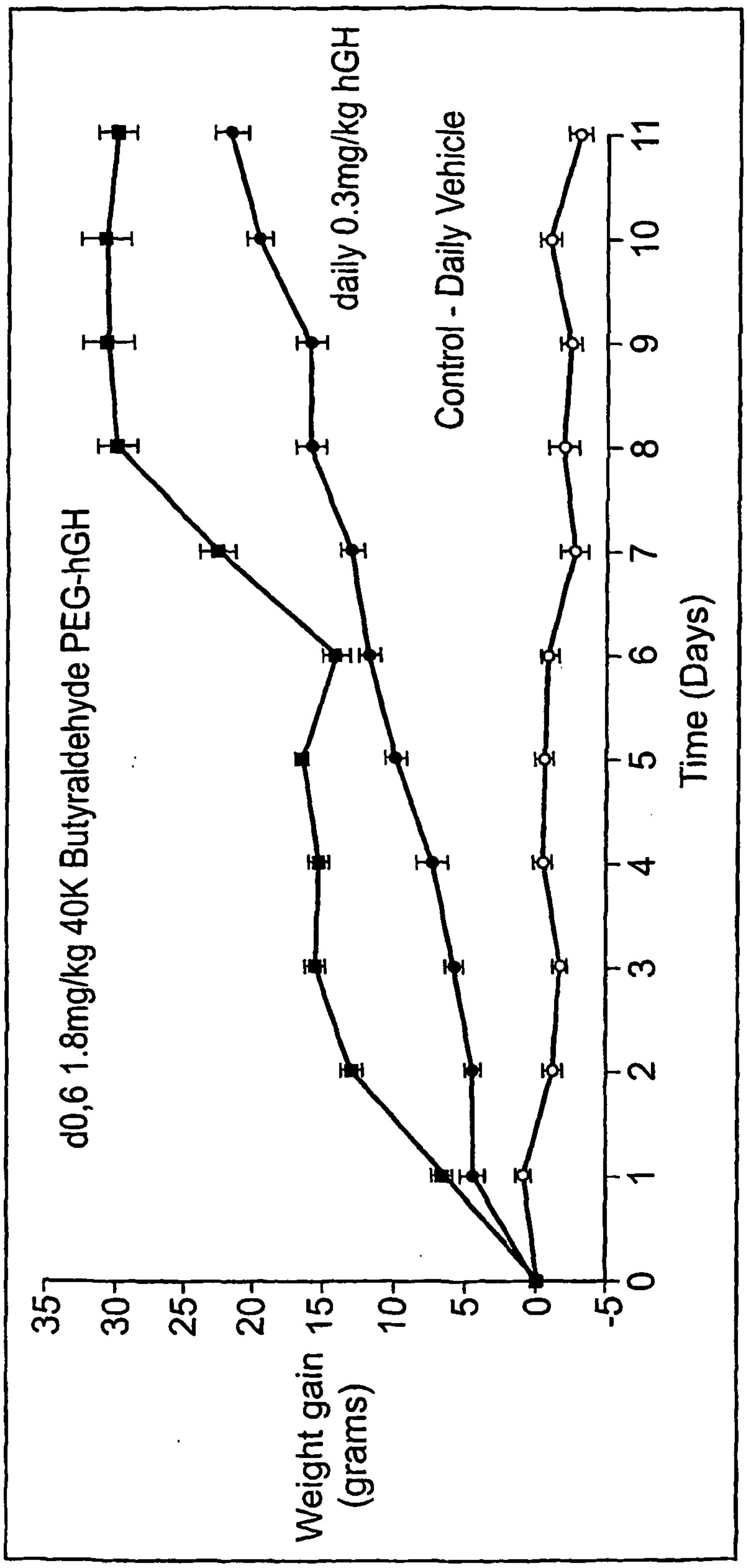
d0,6 1.8mg/kg 40K Butyraldehyde PEG-hGH
daily 0.3mg/kg hGH
Control - Daily Vehicle
Weight gain (grams)
35
30
25
20
15
10
5
0
-5
Time (Days)
0
1
2
3
4
5
6
7
8
9
10
11

FIG. 4

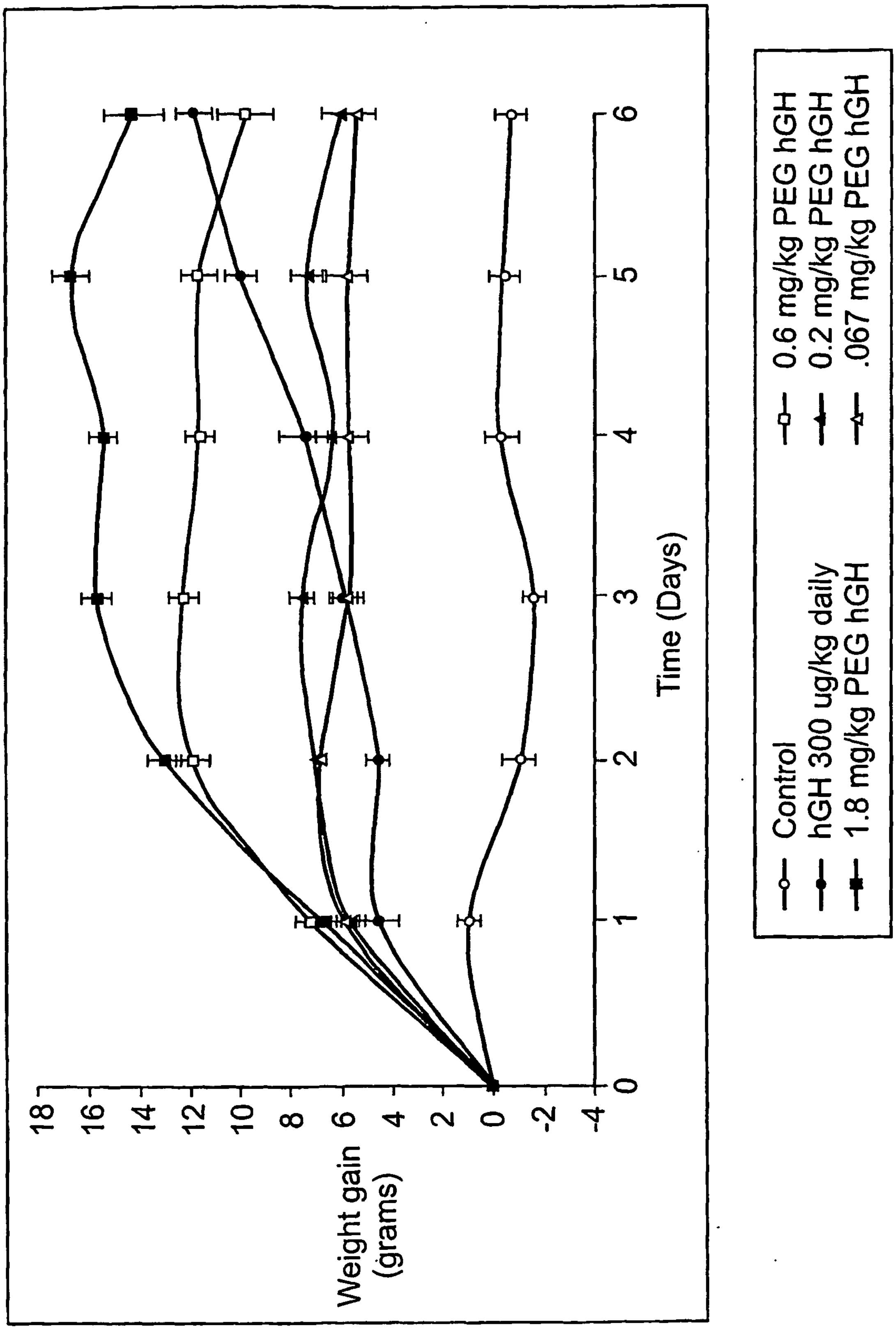
Weight gain
(grams)
18
16
14
12
10
8
6
4
2
0
-2
-4
0
1
2
3
4
5
6
Time (Days)
Control
hGH 300 ug/kg daily
1.8 mg/kg PEG hGH
0.6 mg/kg PEG hGH
0.2 mg/kg PEG hGH
.067 mg/kg PEG hGH

FIG. 5

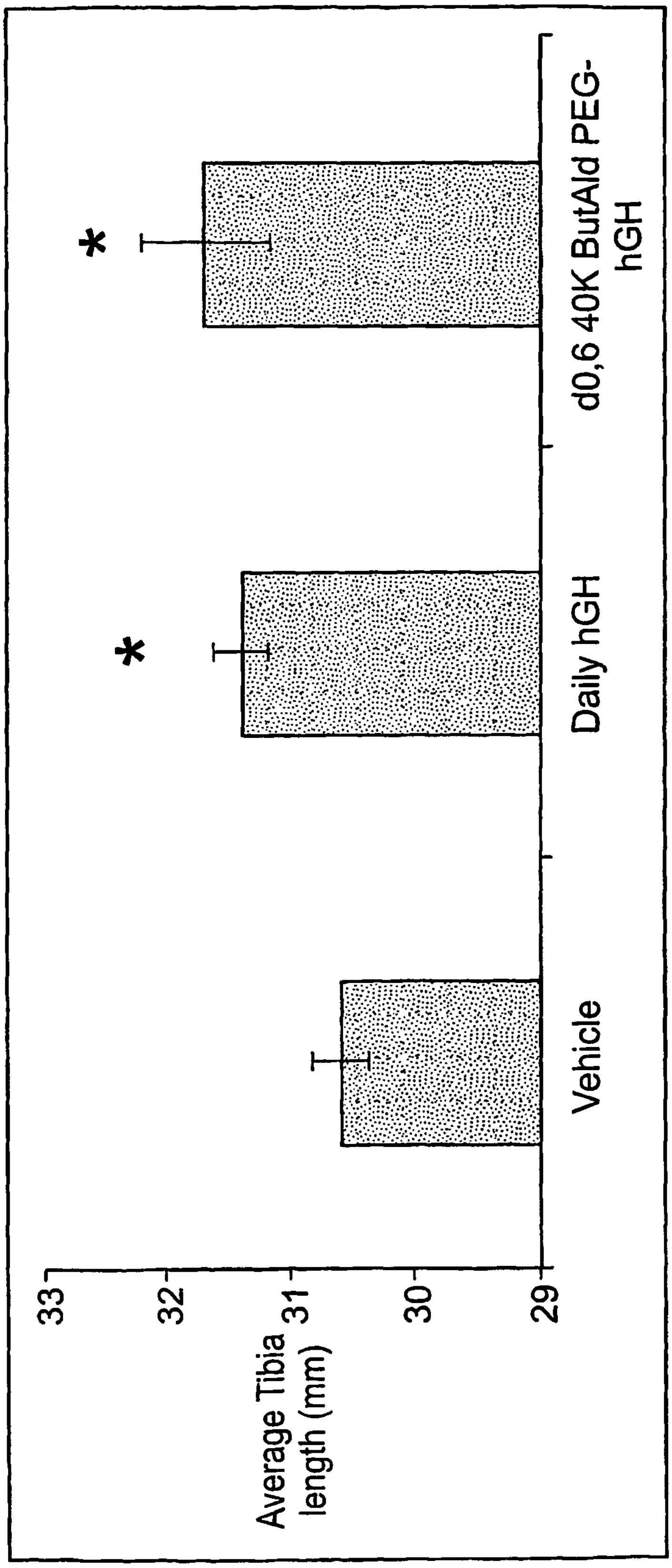
Average Tibia length (mm)
33
32
31
30
29
*
*
Vehicle
Daily hGH
d0,6 40K ButAld PEG-hGH

FIG. 6

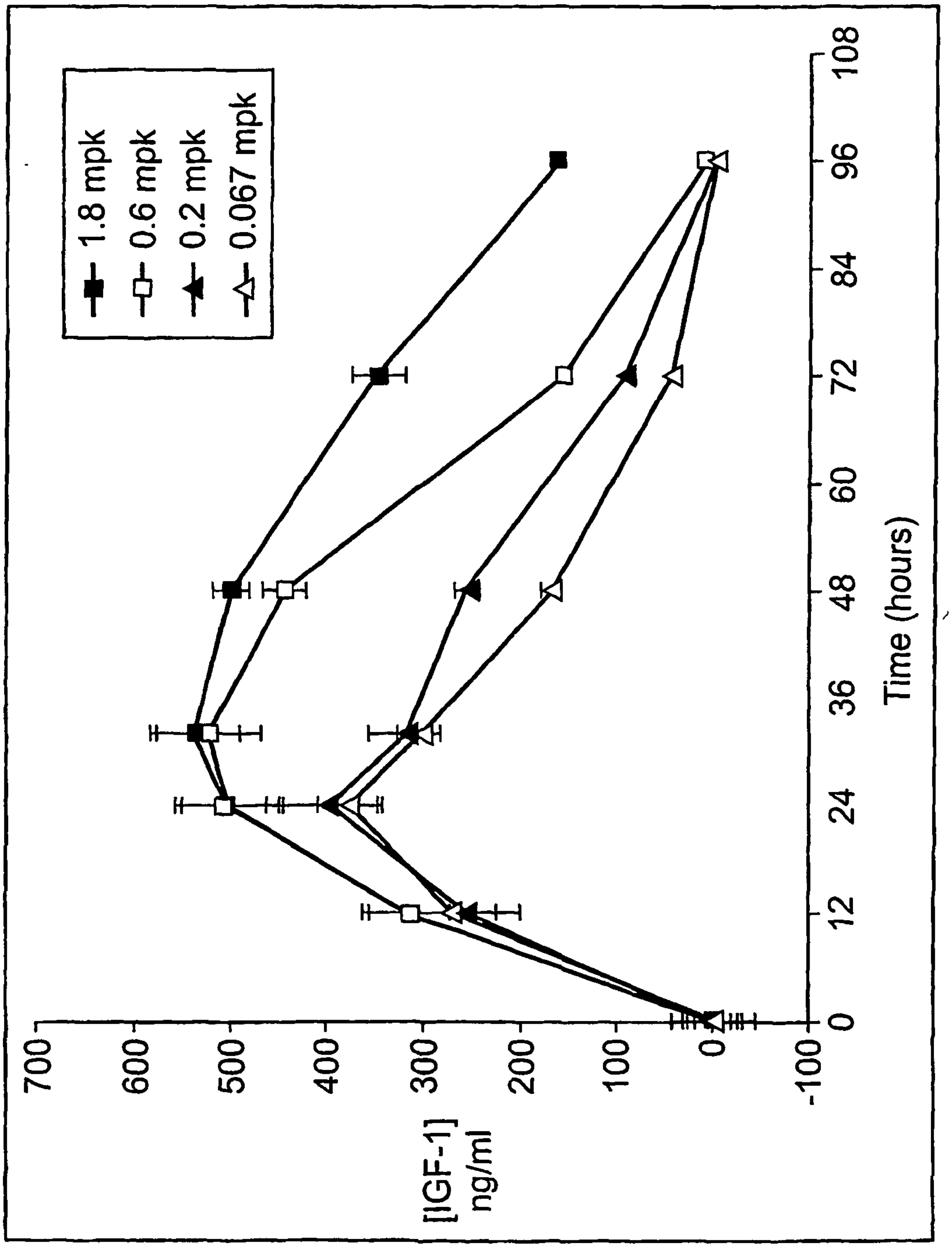
1.8 mpk
0.6 mpk
0.2 mpk
0.067 mpk
[IGF-1]
ng/ml
700
600
500
400
300
200
100
0
-100
0
12
24
36
48
60
72
84
96
108
Time (hours)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10771895 B
- US 4179337 A, Davis
- US 4002531 A, Royer
- US 5738846 A
- US 5382657 A
- EP 0401384 A
- EP 0473268 A
- EP 0335423 A
- US 5824784 A
- US 5824778 A
- US 4904584 A
- WO 9300109 A
- WO 9903887 A
- WO 9420069 A
- EP 458064 A2
- WO 9511987 A
- WO 0042175 A
- WO 9711178 A
- US 5849535 A
- US 6004931 A
- US 6022711 A
- WO 03044056 A
- US 4658021 A
- US 5633352 A
- US 4342832 A
- US 4601980 A
- US 4898830 A
- US 5424199 A
- US 5795745 A
- US 6143523 A
- WO 9209690 A
- US 5478925 A
- US 5073627 A
- WO 9410308 A
- US 463121 A
- WO 8403564 A
- WO 8403506 A
- US 5932462 A
- US 5342940 A
- US 5643575 A
- US 5919455 A
- US 6113906 A
- US 5183660 A
- WO 0209766 A
- WO 0126692 A
- WO 9005534 A
- US 5057417 A
- WO 2002057241 A
- WO 2002056873 A
- US 4411890 A
- WO 8907110 A
- WO 8907111 A
- WO 9304081 A
- WO 9858947 A
- US 6521644 B
- WO 9515309 A
- WO 9819998 A
- US 20030100563 A
- WO 2003082817 A
- US 20030055261 A
- US 20030040483 A
- EP 18072 A
- EP 83864 A
- WO 8901711 A
- WO 8910933 A
- WO 889780 A
- WO 8302272 A
- WO 9118016 A
- WO 9201711 A
- WO 9514666 A
- EP 0923539 A
- US 5206235 A
- US 5283241 A
- US 5284841 A
- US 5310737 A
- US 5317017 A
- US 5374721 A
- US 5430144 A
- US 5434261 A
- US 5438136 A
- US 5494919 A
- US 5494920 A
- US 5492916 A
- US 5536716 A
- US 5578593 A
- WO 9413696 A
- WO 9419367 A
- WO 9503289 A
- WO 9503290 A
- WO 9509633 A
- WO 9511029 A
- WO 9512598 A
- WO 9513069 A
- WO 9516675 A
- WO 9516692 A
- WO 9517422 A
- WO 9517423 A
- WO 9534311 A

- WO 9602530 A
- US 5804578 A
- US 5783582 A
- WO 2004007468 A
- WO 0104119 A
- US 6329383 B
- EP 1155014 A
- WO 0170228 A
- WO 0170227 A
- WO 0069433 A
- WO 0004013 A
- WO 995156 A
- WO 9951595 A
- WO 99512314 A
- WO 99412512 A
- WO 9921557 A
- WO 9921553 A
- WO 0053602 A
- WO 0053185 A
- WO 0053181 A
- WO 0053180 A
- WO 0053179 A
- WO 0053178 A
- US 6288078 B


**Non-patent literature cited in the description**


- **K. BARNEIS. ; U. KELLER.** *Baillieres Clin. Endocrinlo. Metab.,* 1996, vol. 10, 337
- **J. VERHELST J ; R. ABS.** *Drugs,* 2002, vol. 62, 2399
- **NICOLL, C. S. et al.** *Endocrine Reviews,* 1986, vol. 7, 169
- **LEUNG, D. W. et al.** *Nature,* 1987, vol. 330, 537
- **BOUTIN, J. M. et al.** *Cell,* 1988, vol. 53, 69
- **CHANG, C. N. et al.** *Gene,* 1987, vol. 55, 189
- **GOEDDEL et al.** *Nature,* 1979, vol. 281, 544
- **GRAY et al.** *Gene,* 1985, vol. 39, 247
- **CHAWLA, R, K.** *Ann. Rev. Med.,* 1983, vol. 34, 519
- **EDWARDS, C. K. et al.** *Science,* 1988, vol. 239, 769
- **THOMER, M. 0. et al.** *J. Clin. Invest,* 1988, vol. 81, 745
- **NIALL.** *Nature, New Biology,* 1971, vol. 230, 90
- **MOORE et al.** *Endocrinology,* 1988, vol. 122, 2920-2926
- **CHARLES C. THOMAS.** *Springfield,* 1964
- **R. K. CHAWLA et al.** *Annu. Rev. Med.,* 1983, vol. 34, 519
- **0. G. P. ISAKSSON et al.** *Annu. Rev. Physiol.,* 1985, vol. 47, 483
- **C. K. EDWARDS et al.** *Science,* 1988, vol. 239, 769
- **M. 0. THOMER ; M. L. VANCE.** *J. Clin. Invest.,* 1988, vol. 82, 745
- **J. P. HUGHES ; H. G. FRIESEN.** *Ann. Rev. Physiol.,* 1985, vol. 47, 469
- **MILLARD et al.** *Neurobiol. Aging,* 1990, vol. 11, 229-235
- **TAKAHASHI et al.** *Neuroendocrinology M,* 1987, vol. L6, 137-142
- **RUDMAN et al.** *J. Clin. Invest.,* 1981, vol. 67, 1361-1369
- **BLACKMAN.** *Endocrinology and Aging,* 1987, vol. 16, 981
- **RUDMAN et al.** *N. Eng. J. Med.,* 1990, vol. 323, 1-6
- **D. W. LEUNG et al.** *Nature,* 1987, vol. 330, 537
- **J. M. BOUTIN et al.** *Mol. Endocrinology.,* 1989, vol. 3, 1455
- **M. FREEMARK ; M. COMER ; G. KOMER ; S. HANDWERGER.** *Endocrinol,* 1987, vol. 120, 1865
- **SADA et al.** *. Fermentation Bioengineering,* 1991, vol. 71, 137-139
- **ABUCHOWSKI et al.** Enzymes as Drugs. 1981, 367-383
- **CHAMOW et al.** *Bioconjugate Chem.,* 1994, vol. 5, 133-140
- *Journal of Biological Chemistry,* 1996, vol. 271, 21969-21977
- **OLSON et al.** *Polymer Preprints,* 1997, vol. 38, 568-569
- **CLARK et al.** *Journal of Biological Chemistry,* 1996, vol. 271, 21969-21977
- **RON et al.** *Biol Chem.,* 1993, vol. 268, 2984-2988
- **BOWIE et al.** *Science,* 1990, vol. 247, 1306-1310
- **HOFMANN et al.** *Nucl. Acids Res.,* 1999, vol. 27, 215-219
- **BUCHER ; BAIROCH et al.** Proceedings 2nd International Conference on Intelligent Systems for Molecular Biology. AAAIPress, 1994, 53-61
- **SONNHAMMER et al.** *Proteins,* 1997, vol. 28 (3), 405-20
- **HENIKOFF et al.** *Nucleic Acids Res.,* 2000, vol. 28 (1), 228-30
- **BATEMAN et al.** *Nucleic Acids Res.,* 2000, vol. 28 (1), 263-6
- **HENIKOFF et al.** *Electrophoresis,* 2000, vol. 21 (9), 1700-6
- **ATTWOOD et al.** *Nucleic Acids Res.,* 1996, vol. 24 (1), 182-8
- **SONNHAMMER ; KAHN.** *Protein Sci.,* 1994, vol. 3 (3), 482-92

- **CORPET et al.** *Nucleic Acids Res.,* 2000, vol. 28 (1), 267-9
- **PONGOR et al.** *Protein Eng.,* 1993, vol. 6 (4), 391-5
- **MURVAI et al.** *Nucleic Acids Res.,* 2000, vol. 28 (1), 260-2
- **SCHULTZ et al.** *Proc. Natl. Acad. Sci. U S A,* 1998, vol. 95, 5857-5864
- **HOLM ; SANDER.** *Nucleic Acids Res.,* 1996, vol. 24 (1), 206-9
- **HOLM ; SANDER.** *Nucleic Acids Res.,* 1997, vol. 25 (1), 231-4
- **HOLM ; SANDER.** *Nucleic Acids Res.,* 1999, vol. 27 (1), 244-7
- **SANDER ; SCHNEIDER.** *Proteins,* 1991, vol. 9 (1), 56-68
- **ORENGO et al.** *Structure,* 1997, vol. 5 (8), 1093-108
- **PEARL et al.** *Biochem. Soc. Trans.,* 2000, vol. 28 (2), 269-75
- **MURZIN et al.** *J. Mol. Biol.,* 1995, vol. 247 (4), 536-40
- **LO CONTE et al.** *Nucleic Acids Res.,* 2000, vol. 28 (1), 257-9
- **TATUSOV et al.** *Science,* 1997, vol. 278, 631-637
- **TATUSOV et al.** *Nucleic Acids Res.,* 2000, vol. 28 (1), 33-6
- **NEVILL-MANNING et al.** *Proc. Natl. Acad. Sci. USA.,* 1998, vol. 95, 5865-5871
- **YONA et al.** *Proteins,* 1999, vol. 37 (3), 360-78
- **ATTWOOD et al.** *Nucleic Acids Res.,* 2000, vol. 28 (1), 225-7
- *Nucleic Acid Research,* 2000, vol. 28 (1
- **HOUGHTEN.** *Proc. Natl. Acad. Sci. USA,* 1985, vol. 82, 5131-5135
- **GEYSEN et al.** *Proc. Natl. Acad. Sci. U.S.A.,* 1984, vol. 81, 3998-4002
- **JAMESON ; WOLF.** *Comp. Appl. Biosci.,* 1988, vol. 4, 181-186
- Methods in Enzymology. Academic Press, 1993
- **CREIGHTON.** Proteins: Structures and Molecular Principles. 1983
- **HUNKAPILLER et al.** *Nature,* 1984, vol. 310 (5973), 105-11
- **DAVIS et al.** Basic Methods in Molecular Biology. Elsevier Press, 1986
- **PEARSON ; LIPMAN.** *Proc. Natl. Acad. Sci. USA,* 1988, vol. 85, 2444-2448
- **ALTSCHUL et al.** *J. Mol. Biol.,* 1990, vol. 215, 403-410
- **ALTSCHUL et al.** *Nature hGHtics,* 1993, vol. 3, 266-272
- **ALTSCHUL et al.** *Nuc. Acids Res.,* 1997, vol. 25, 3389-3402
- **THOMPSON et al.** *Nuc. Acids Res.,* 1994, vol. 22, 4673-4680
- **HIGGINS et al.** *Meth. Enzymol.,* 1996, vol. 266, 383-402
- **BRUTLAG et al.** *Comp. App. Biosci.,* 1990, vol. 6, 237-245
- **JONES ; SWINDELLS.** *Trends Biochem Sci,* 2002, vol. 27, 161-4
- **OLSEN et al.** *Biocomput,* 1999, vol. 302, 13
- **GONNET et al.** *Science,* vol. 256, 1443-1445
- **HENIKOFF ; HENIKOFF.** *Proteins,* 1993, vol. 17, 49-61
- Matrices for Detecting Distance Relationships: Atlas of Protein Sequence and Structure. National Biomedical Research Foundation, 1978
- **ROBERTS M.J. et al.** *Adv. Drug Del. Rev.,* 2002, vol. 54, 459-476
- **HARRIS J.M. et al.** *Drug Delivery Sytems,* 2001, vol. 40, 538-551
- **KODERA Y.** *Bioconjugate Chemistry,* 1994, vol. 5, 283-288
- **CLARK et al.** *Journal of Biological Chemistry,* vol. 271, 21969-21977
- **NEUGEBAUER.** *A Guide to the Properties and Uses of Detergents in Biology and Biochemistry,* 1992